# EUROPÄISCHE PATENTSCHRIFT

(11) **EP 0 195 947 B1**
(45) Veröffentlichungstag und Bekanntmachung des Hinweises auf die Patenterteilung: **24.04.1996**
(21) Anmeldenummer: 86102491.7
(22) Anmeldetag: 26.02.1986
(51) Int. Cl.: C07D 501/20, C07D 501/59, C07D 499/68, C07D 499/70, A61K 31/545, A23K 1/17, A61K 31/45, C07D 499/00

(54) **Beta-Lactamantibiotika, Verfahren zur Herstellung und ihre Verwendung als und in Arzneimitteln**
Beta-lactam antibiotics, process for their preparation and their use as and in medicaments
Bêta-lactame antibiotiques, leur procédé de préparation et leur application dans des médicaments

(30) Priorität: 08.03.1985 DE 3508258
(43) Veröffentlichungstag der Anmeldung: 01.10.1986
(73) Patentinhaber: BAYER AG, D-51368 Leverkusen (DE)
(72) Erfinder: Schmidt, Gunter, Dr., D-5600 Wuppertal 1 (DE); Zeiler, Hans-Joachim, Dr., 5600 Wuppertal 1 (DE); Metzger, Karl Georg, Dr., D-5600 Wuppertal 1 (DE)

(56) Entgegenhaltungen:
- EP-A- 0 122 158
- FR-A- 2 418 232
- FR-A- 2 418 234
- GB-A- 2 015 512
- A.Burger "Medicinal Chemistry", John Wiley and Sons, Inc., New York, 1951, Seiten 75-77
- C.R. Craig and R.E. Stitzel "Modern Pharmacology", Little Brown and Co., Boston, 1986, Seiten 674-68'

## Beschreibung

Die Erfindung betrifft β-Lactamantibiotika, Verfahren zu ihrer Herstellung sowie ihre Verwendung als und in Arzneimitteln, insbesondere als antibakterielle, oral wirksame Antibiotika.

Es ist bekannt, daß verschiedene Vertreter der 7-α-Aminoacylcephalosporine mit unterschiedlichen Substituenten in der 3-Stellung des Moleküls antibiotisch wirken, so zum Beispiel Cephalexin[7-(D-α- Phenylglycylamido)-3-methyl-3-cephem-4-carbonsäure], Cefaclor [7-(D-α-Phenylglycylamido)-3-chlor-3-cephem-4- carbonsaure] (vgl. GB-Patent 1 174 335; Deutsche Offenlegungsschrift 24 08 698 und 27 28 578).

Die vorliegende Erfindung betrifft β-Lactamverbindungen der allgemeinen Formel (I) 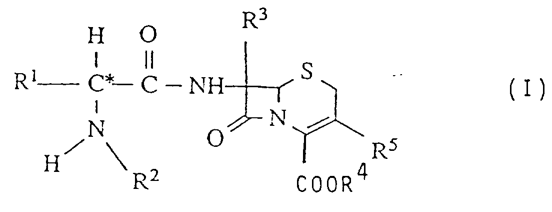 worin
- R⁵: - für Wasserstoff,
- für Fluor, Chlor, Brom,
- für geradkettiges oder verzweigtes, gesättigtes oder ungesättigtes Alkyl mit bis zu 5 C-Atomen steht, das gegebenenfalls substituiert ist durch ein oder mehrere Fluor, Chlor, Brom, Alkoxy, Alkylthio mit jeweils 1 bis 3 C-Atomen Carbamoyloxy, Acetyloxy, Benzoyloxy, oder durch einen Rest der Formel 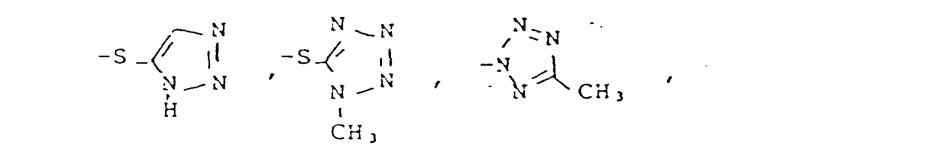 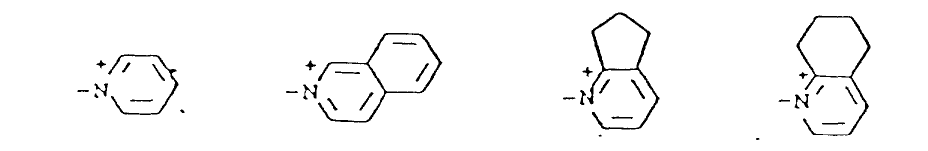 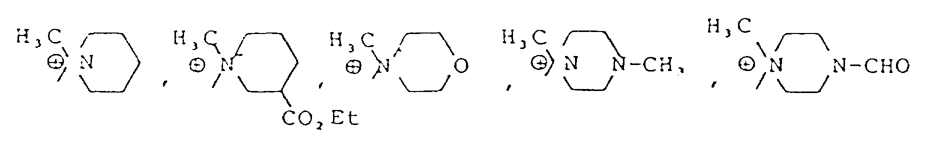 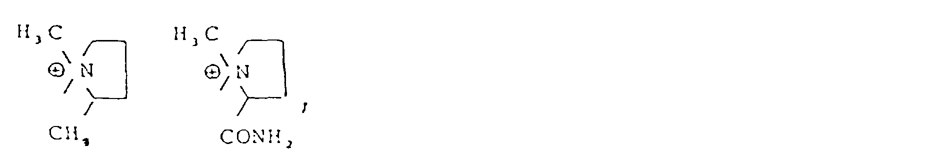 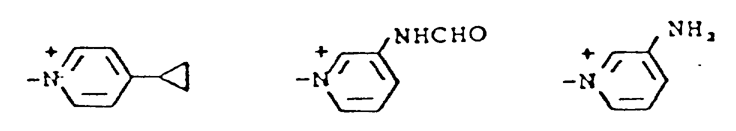 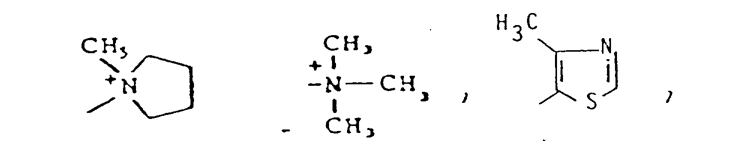
- R¹: für einen Rest der Formel 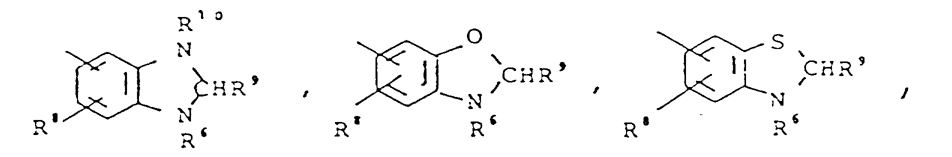 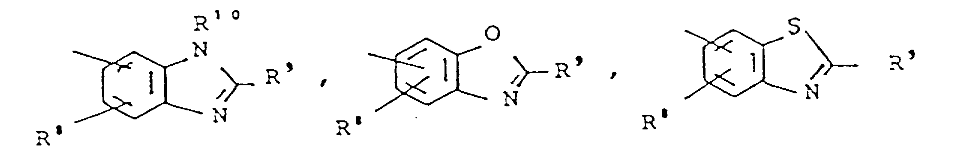 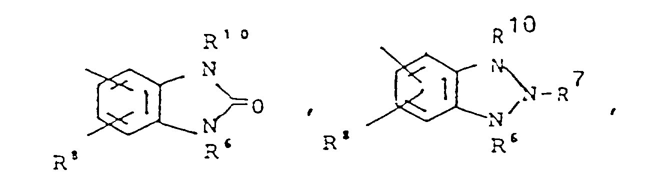 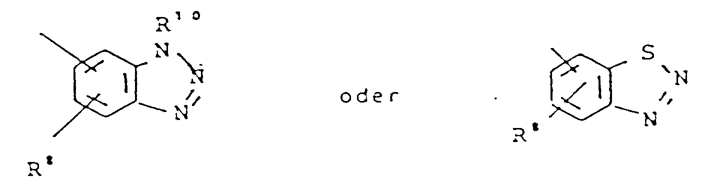
steht,
wobei
- R⁶: - für Wasserstoff,
- für Hydroxy oder Amino steht, oder
- für geradkettiges, verzweigtes oder cyclisches,
   gesättigtes oder ungesättigtes, gegebenenfalls durch ein oder mehrere Fluor, Chlor, Brom, Amino, Hydroxy oder Phenyl substituiertes Alkyl mit bis zu 8 C-Atomen steht, oder
- für Phenyl steht,
- R⁷: - für Wasserstoff, oder
- für geradkettiges, verzweigtes oder cyclisches, gesättigtes oder ungesättigtes Alkyl mit bis zu 8 C-Atomen steht, das gegebenenfalls substituiert ist durch ein oder mehrere Fluor, Chlor, Brom, C₁-C₄-Alkoxy, Hydroxy, Carboxy, Phenyl, SO₃H oder eine Aminogruppe, oder
- für Phenyl steht,
- R⁸: - für Wasserstoff,
- für Alkyl, Alkoxy, Alkylthio mit jeweils 1 bis 6 C-Atomen,
- für Trifluormethyl, Trifluormethoxy,
- für Hydroxy, Mercapto, Nitro, Cyano,
- für Fluor, Chlor, Brom oder
- für eine Aminogruppe steht,
und wobei
- R⁹: - die gleiche Bedeutung hat wie R⁷ und außerdem
- für Fluor, Chlor, Brom,
- für C₁-C₆-Alkoxy, C₁-C₆-Alkylthio steht,
- für eine Aminogruppe,
- für -SO₂-C₁-C₆-Alkyl, -PO(OH)₂,
- für -SO₃H oder -SO₂NH₂ steht,
- für SH, OH, S-Phenyl oder O-Phenyl,
- für Guanidino, -NHNH₂, NHOH, oder
- für ein gegebenenfalls ein- bis zweifach gleich oder verschieden durch Alkyl, Alkylthio, Alkoxy mit jeweils 1 bis 2 C-Atomen,
   Fluor, Chlor, Brom, Nitro, Cyano, Hydroxy, Amino, Trifluormethyl, Trifluormethoxy oder Trifluormethylthio substituiertes Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxalyl, Chinazolyl, Piperidinyl, Oxazolyl, Thiazolyl, Isoxazolyl, Thiadiazolyl, Triazolyl oder Tetrazolyl steht,
- R¹⁰: - die gleiche Bedeutung hat wie R⁶, jedoch nicht mit R⁷ eine Doppelbindung vervollständigt,
oder
- R⁹ und R¹⁰: gemeinsam für eine gegebenenfalls durch Schwefel unterbrochene C₂-C₄-Methylenkette stehen,

- R: - für Wasserstoff oder
- für eine leicht abspaltbare Aminoschutzgruppe steht,
- R³: - für Wasserstoff,
- für Alkoxy, Alkylthio mit jeweils 1 bis 3 C-Atomen,
- für eine Aminogruppe oder
- für NHCHO steht,
und
- R⁴: - für Wasserstoff,
- für eine in der β-Lactamchemie übliche Carboxyschutzgruppe,
- für -CH₂-O-CO-C(CH₃)₃,
- für -CH(CH₃)-O-CO-O-C₂H₅, -CH₂-O-CO-CH₃,
- für den Rest der Formel 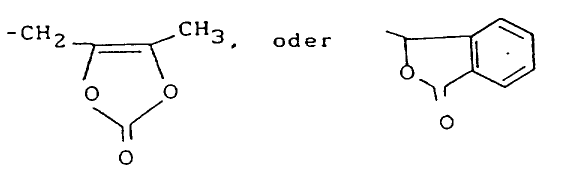 oder
- für Na⁺, Li⁺, K⁺ oder NH₄ ⁺ steht.

Wenn R für eine Aminoschutzgruppe steht, dann bevorzugt fur eine leicht abspaltbare Aminoschutzgruppe wie z.B. tert.-Butoxycarbonyl (Boc), Trityl (Trt), Benzyloxycarbonyl (Z), Formyl, Chloracetyl oder 1-Methyl-2-ethoxycarbonyl-vinyl.

Wenn R⁴ eine Carboxyschutzgruppe ist, dann bevorzugt eine in der β-Lactamchemie übliche Schutzgruppe, bevorzugt tert.-Butyl, Decyl, 2,2,2-Trichlorethyl, Benzyl, 4-Methoxybenzyl, 4-Nitrobenzyl, Tri- bzw. Diphenylmethyl , Acetoxymethyl, Allyl oder Trimethylsilyl.

Die Verbindungen der Formel I können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium- oder Ammoniumsalze und nicht-toxische substituierte Ammoniumsalze, mit Aminen wie Di-, Tri-niedrigalkylaminen, Procain, Dibenzylamin, N,N'-Dibenzylethylendiamin, N-Benzyl-β-phenyl-ethylamin, N-Methyl und N-Ethylmorpholin, 1-Ephenamin, Dehydroabietylamin, N,N'-Bis-dehydroabietylethylendiamin, N-Niedrigalkylpiperidin und anderen Aminen, die zur Bildung von Salzen von Penicillinen und Cephalosporinen verwendet werden können, vorliegen.

Wegen der Anwesenheit des mit * bezeichneten asymmetrischen Kohlenstoffatoms schließen die neuen β-Lactamantibiotika der Formel I die D-, L- und D,L-Formen ein. Bevorzugt sind die D-Formen der erfindungsgemäßen Verbindungen der allgemeinen Formel I.

Sowohl die Diastereomerengemische als auch die D-Form und L-Form der erfindungsgemäßen Verbindungen können zur Behandlung bakterieller Infektionskrankheiten eingesetzt werden.

Die Verbindungen der allgemeinen Formel I erhält man, wenn man Verbindungen der allgemeinen Formel IIa 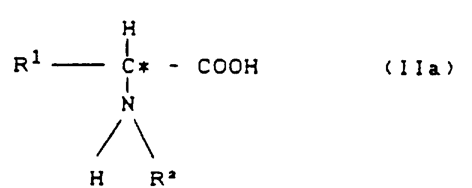 in welcher R¹ die oben angegebene Bedeutung hat und in welcher R für eine Aminoschutzgruppe steht, nach Aktivierung der Carboxylgruppe durch Überführung in ein gemischtes Anhydrid, beispielsweise mit Pivaloylchlorid, Chlorameisensäureethyl- oder isobutylester, nach Überführung in das Mesylat mittels Methansulfonsäurechlorid oder nach Überführung in einen aktivierten Ester, z.B. mit 1-Hydroxybenztriazol und Dicyclohexylcarbodiimid, mit Verbindungen der allgemeinen Formel III 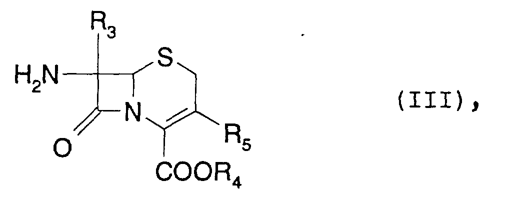 in welcher R³, R⁴ und R⁵ die oben angegebene Bedeutung haben, zur Umsetzung gebracht werden, dann gegebenenfalls Schutzgruppen abgespalten werden und die gewünschten Salze oder aus Salzen die freien Säuren hergestellt werden.

Für die Kupplung der Aminosäuren der Formel II an β-Lactame der Formel III kann eine große Zahl von aus der Cephalosporinchemie bekannten Methoden verwendet werden.

Es hat sich als vorteilhaft erwiesen, Aminosäuren der allgemeinen Formel II zu aktivieren und dann mit β-Lactamen der allgemeinen Formel III, die als Salze mit einem Amin in Lösung gebracht wurden, zu kuppeln.

Besonders vorteilhaft ist die Aktivierung mit Pivalinsäurechlorid oder Sulfonsäurederivaten der Formel IV zu Anhydriden der Formel Va und Vb. 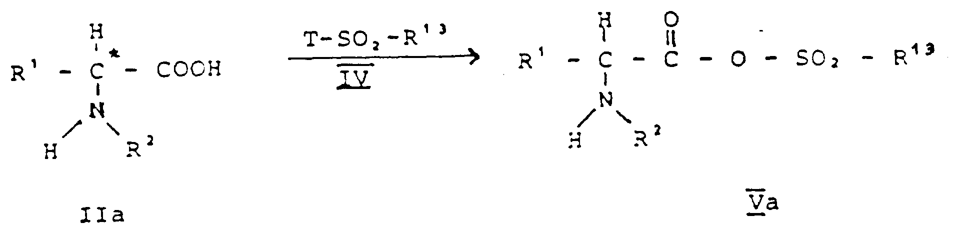 oder 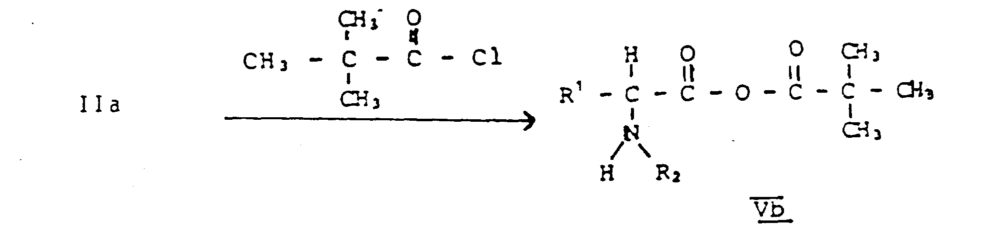 in welchen
- R¹ -: die oben angegebene Bedeutung hat,
- R -: für eine Aminoschutzgruppe steht,
- T -: für einen Rest R¹³-SO₂-O oder Halogen steht, und
- R¹³-: für C₁-C₁₀-Alkyl steht, das gegebenenfalls substituiert ist durch Fluor, Chlor, Cyano, Phenyl, C₁-C₄-Alkyl, C₁-C₄-Alkoxy oder C₁-C₄-Alkylthio, oder
- für gegebenenfalls durch Fluor, Chlor, Brom, Cyano, C₁-C₄-Alkyl, C₁-C₄-Alkoxy, C₁-C₄-Alkylthio, C₁-C₄-Alkylcarbonyl, Nitro, Trifluormethyl substituiertes Phenyl steht.

Wenn R¹³ substituiert ist, sind vorzugsweise 1-3 Substituenten, vorzugsweise die genannten vorhanden.

Ganz besonders bevorzugt stellt R¹³ einen Methyl- oder p-Tolylrest dar.

Die gemischten Anhydride der Formel Va werden hergestellt, indem man die Säuren der Formel II und 1 bis 1,4-Äquivalente eines Amins in einem Lösungsmittel löst und mit 1 bis 1,2 Äquivalenten eines Sulfonsäurederivates der Formel IV reagieren läßt.

Als Lösungsmittel eignen sich alle Solventien, die unter den Reaktionsbedingungen stabil sind, wie z.B. Diethylether, Tetrahydrofuran, Acetonitril, Aceton, Methylenchlorid, Chloroform oder Dimethylformamid.

Als Amin eignen sich tertiäre Amine wie z.B. Triethylamin oder Tributylamin, aber auch sterisch gehinderte sekundäre Amine, wie z.B. Diisopropylamin.

Die Umsetzungen können bei Temperaturen zwischen -80°C und Raumtemperatur durchgeführt werden, bevorzugt zwischen -60°C und 0°C. Vorteilhaft wird die Aktivierung mit Cl-SO₂-CH₃ in Dimethylformamid bei -40°C bis -60°C innerhalb von 0,2 bis 24 Stunden, vorzugsweise 0,5 bis 5 Stunden, durchgeführt.

Zum Lösen der Verbindungen der Formel III können die bei der Herstellung der Verbindungen der Formel V genannten Lösungsmittel sowie als Base die dort genannten Amine verwendet werden.

Besonders vorteilhaft ist auch eine Aktivierung der Säuren der allgemeinen Formel II durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel V eignen.

Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit einer Verbindung der Formel III in Form einer Lösung ihres Aminsalzes innerhalb von 2 bis 24 Stunden umgesetzt. Zum Lösen der Verbindungen der Formel III können die bei der Herstellung der Verbindungen der Formel V genannten Lösungsmittel sowie als Base die dort genannten Amine verwendet werden.

Literatur für Amino- und Carboxylschutz und Carboxyl-Aktivierung: M. Bodanszky, Principles of Peptide Synthesis, Springer-Verlag, 1984. E. Gross, J. Meienhofer, The Peptides Vol. 2, Academic Press, 1980.

Die stereochemish einheitlichen D- bzw. L-Formen der erfindungsgemaßen Verbindungen der Formel I erhält man, wenn man die Diastereomerengemische beispielsweise an HPLC-Säulen von Merck, DuPont oder Whatman trennt.

Andererseits erhält man die reine D- bzw. L-Form (bevorzugt die D-Form), wenn man bereits auf der Stufe der racemischen Aminosäure der Formel II eine chemische Racematspaltung, z.B. mit Dehydroabietylamin, Phenylethylamin oder Camphersulfonsäure, oder eine Racemat-Spaltung z.B. über N-Acetyl-aminosäurederivate, z.B. mit Subtilisin, Penicillinacylase oder Schweinenierenacylase, vornimmt und anschließend die stereochemisch einheitlichen D- bzw. L-Formen der Verbindungen der Formel II in angegebener Weise umsetzt.

Die Verbindungen der allgemeinen Formel II sind nur zum Teil bekannt. Die Verbindungen der Formel II können nach literaturbekannten Verfahren, wie es im Schema 1 dargestellt wird, synthetisiert werden, wobei die Verbindungen der Formel VI die wichtigsten Schlüsselverbindungen für die neue Aminosäuren der Formel II darstellen. 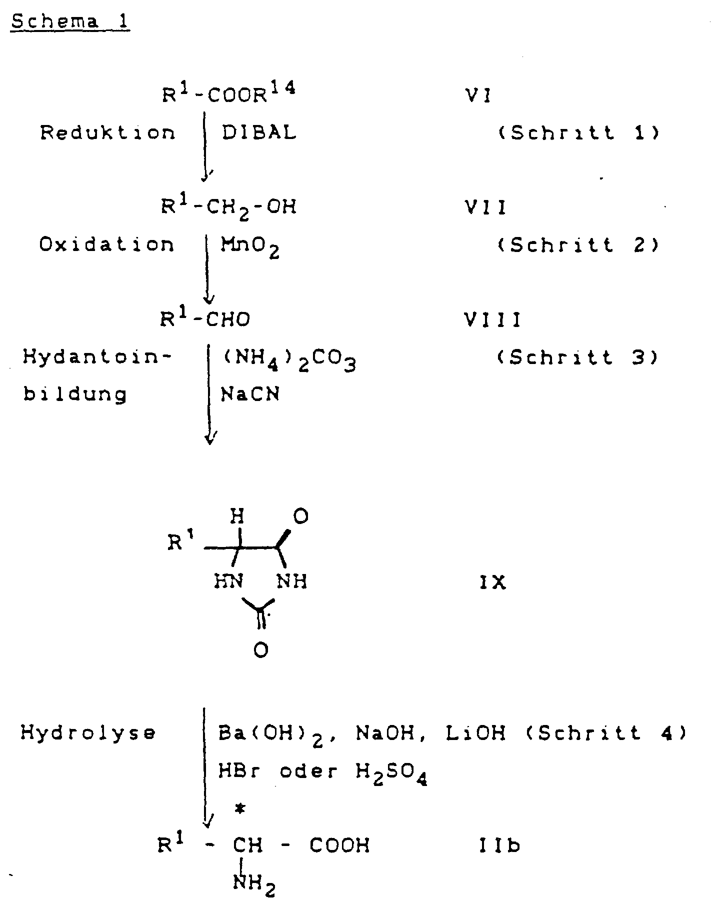
R¹ hat die oben angegebene Bedeutung,
R¹⁴ steht für C₁-C₄-Alkyl,

Die Reduktion von Estern mit Diisobutylaluminiumhydrid (DIBAL) und Natrium-bis(2-methoxyethoxy)aluminiumhydrid (Red-Al) zu Alkoholen (Schritt 1) ist in der Literatur beschrieben: E. Winterfeld, Synthesis 1975, 617; A.E.G. Miller et al., J. Org. Chem. 24, 627 (1959).
Die Oxidation primärer Alkohole mit Mangan(IV)-oxid oder Pyridiniumchromat zu Aldehyden (Schritt 2) ist in der Literatur bekannt:
Methoden der Organischen Chemie, Houben-Weyl, Bd. 4/1b; G. Piancatelli et al., Synthesis 1982, 245.

Die neuen Aminosäuren der Formel IIb erhält man, wenn man die Aldehyde mit Natriumcyanid und Ammoniumcarbonat nach literaturbekannten Verfahren [E. Ware, Chemical Reviews 46, 403, (1950)] umsetzt (Schritt 3) und anschließend mit 10%iger Natronlauge, 48%iger Bromwasserstoffsäure, Bariumhydroxid- oder Lithiumhydroxidlösung hydrolysiert (Schritt 4).

Im folgenden wird beispielhaft die Darstellung einiger neuer Aminosäuren der allgemeinen Formel II bzw. deren Vorstufen beschrieben, wobei R⁹-R¹⁴ die oben angegebene Bedeutung haben:

### 1) Benzthiazolyl-glycine:

Ausgangsmaterial für die Synthese von substituierten Benzthiazolyl-glycinderivaten ist o-, m- oder p-Aminobenzoesäureester. Benzthiazol-carbonsäurederivate werden beispielsweise nach folgendem Syntheseschema hergestellt: 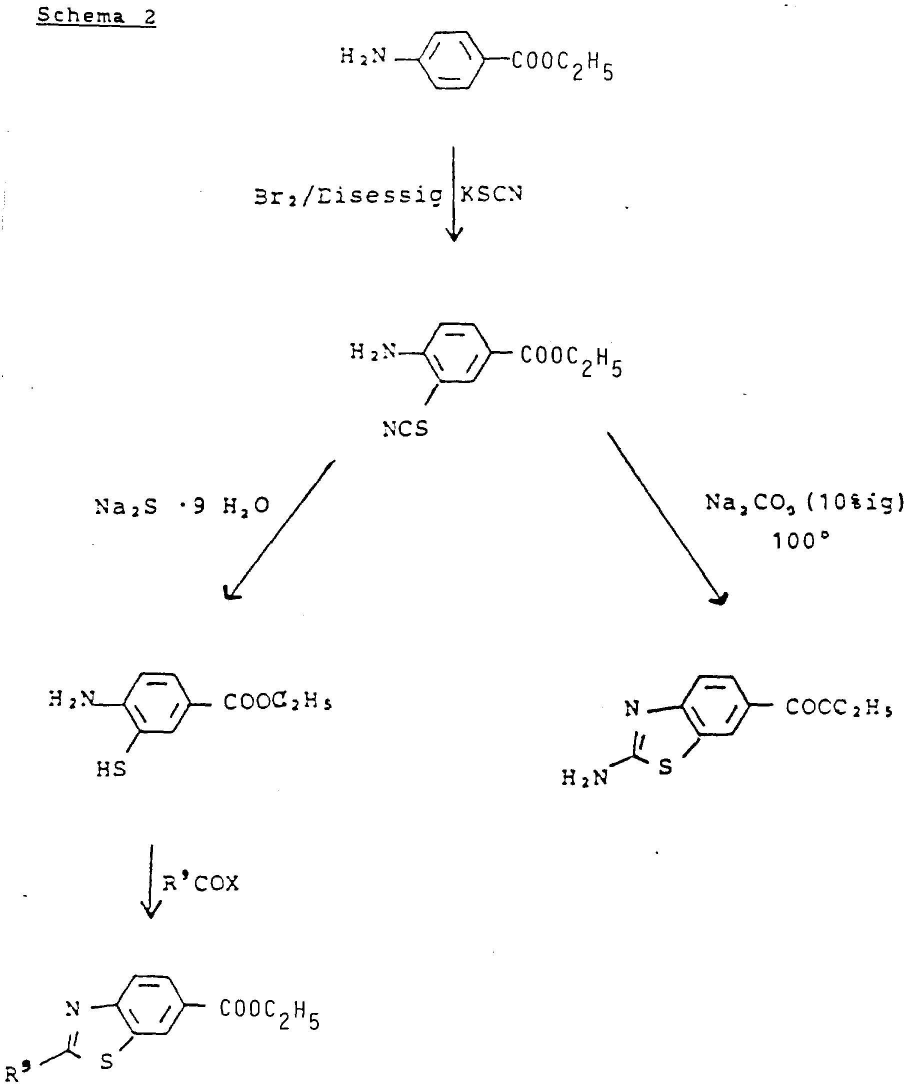 Literatur: J.L. Wood, Substitution and Addition Reactions of Thiocyanogen, Organic Reactions, Vol. III, 240 (1946); M.T. Bogert et al., J. Am. Chem. Soc. 47, 3078 (1925); M.T. Bogert, J. Am. Chem. Soc. 57, 1529 (1935); J. M. Spraque et al., Thiazoles and Benzothiazoles, Heterocyclic Compounds, Vol. 5, 484 (1957), J. Wiley & Sons. Ausgangsmaterial für die Synthese von substituierten Benzthiazolyl-glycin-derivaten ist auch α-Amino-α-(p-aminophenyl)-essigsäureester:

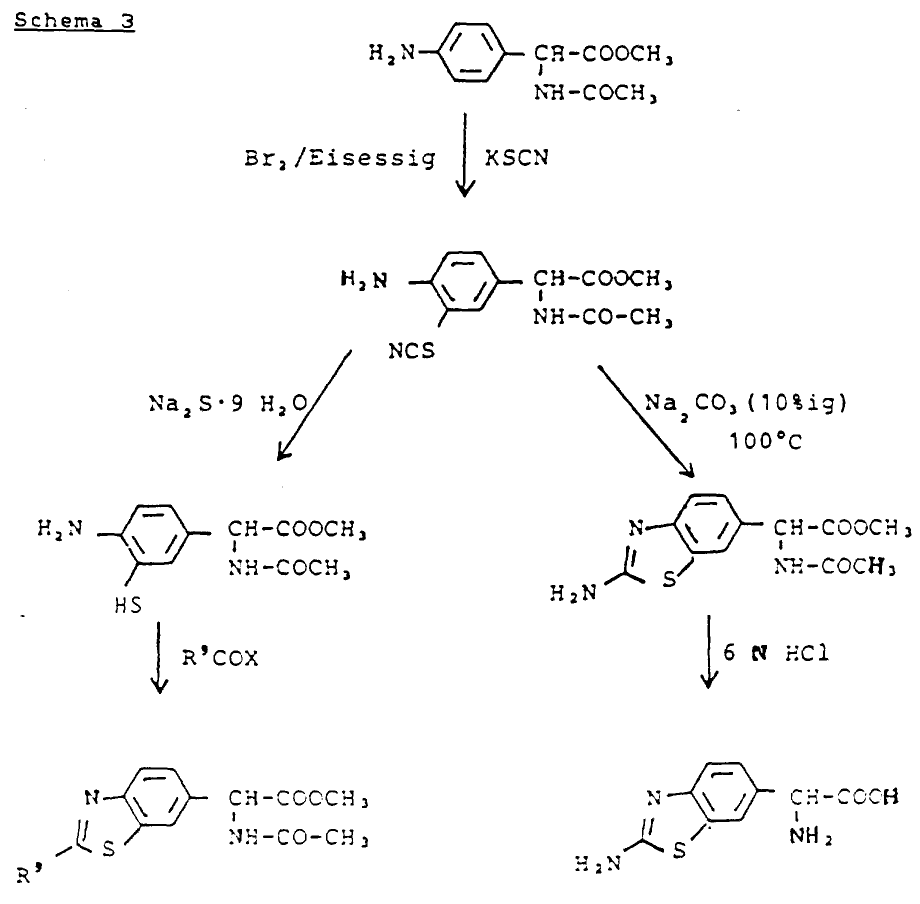

### 2) Benzimidazolyl-glycine

Ausgangsmaterial für die Synthese von substuierten Benzimidazolyl-glycinderivaten ist z.B. 3.4-Diaminobenzoesäure. Die substituierten Benzimidazolcarbonsäureester werden beispielsweise nach folgendem Syntheseschema dargestellt: 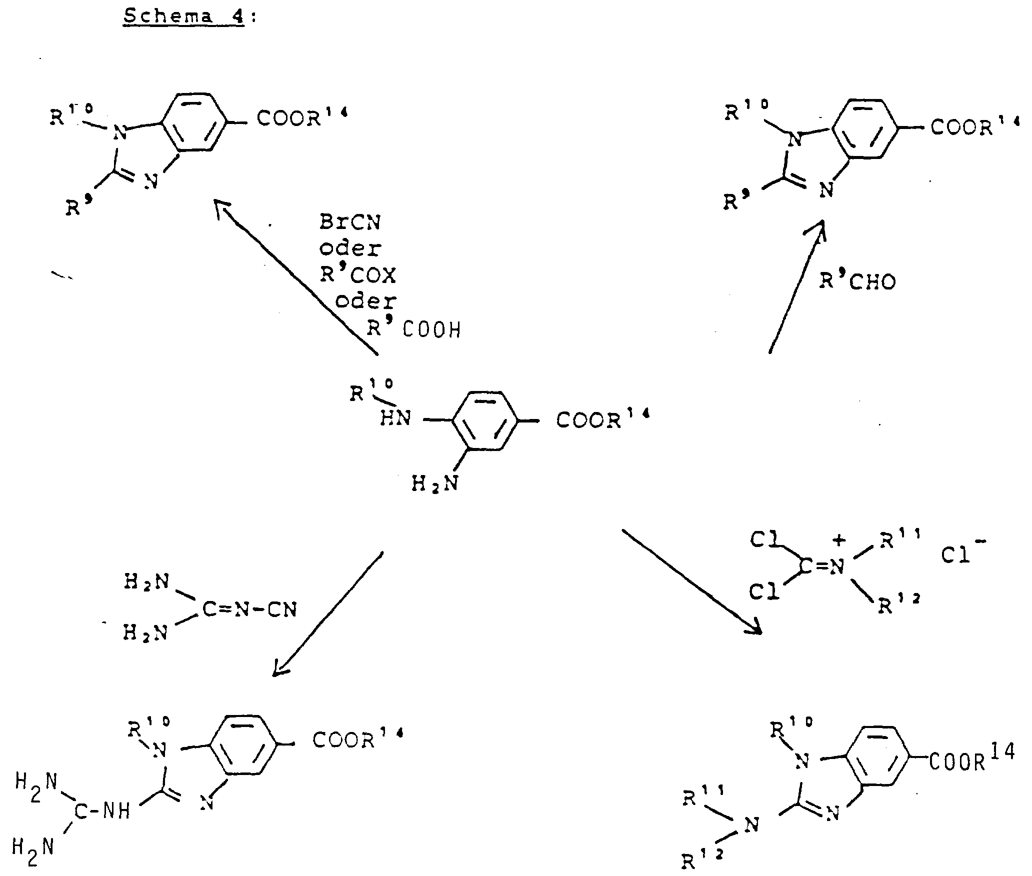 Literatur: P.N. Preston, Chemical Reviews 74, 279 (1974); R. Rastogi et al., Synthesis 1983, 861; P.N. Preston, Benzimidazoles and Congeneric Tricyclic Compounds, Heterocyclic Compounds, Vol. 40, I and II, J. Wiley & Sons (1980, 1981).

### 3) Benzoxazolyl-glycine

Für die Synthese von substituierten Benzoxazolyl-glycinderivaten verwendet man als Ausgangsmaterial 3-Amino-4-hydroxybenzoesäure, 3-Hydroxy-4-aminobenzoesäure und D-α-Amino-(3-amino-4-hydroxyphenyl)essigsäuremethylester. Die neuen anellierten Phenylglycinderivate und substituierten Benzoxazolcarbonsäurederivate werden beispielsweise nach folgendem Syntheseschema dargestellt: 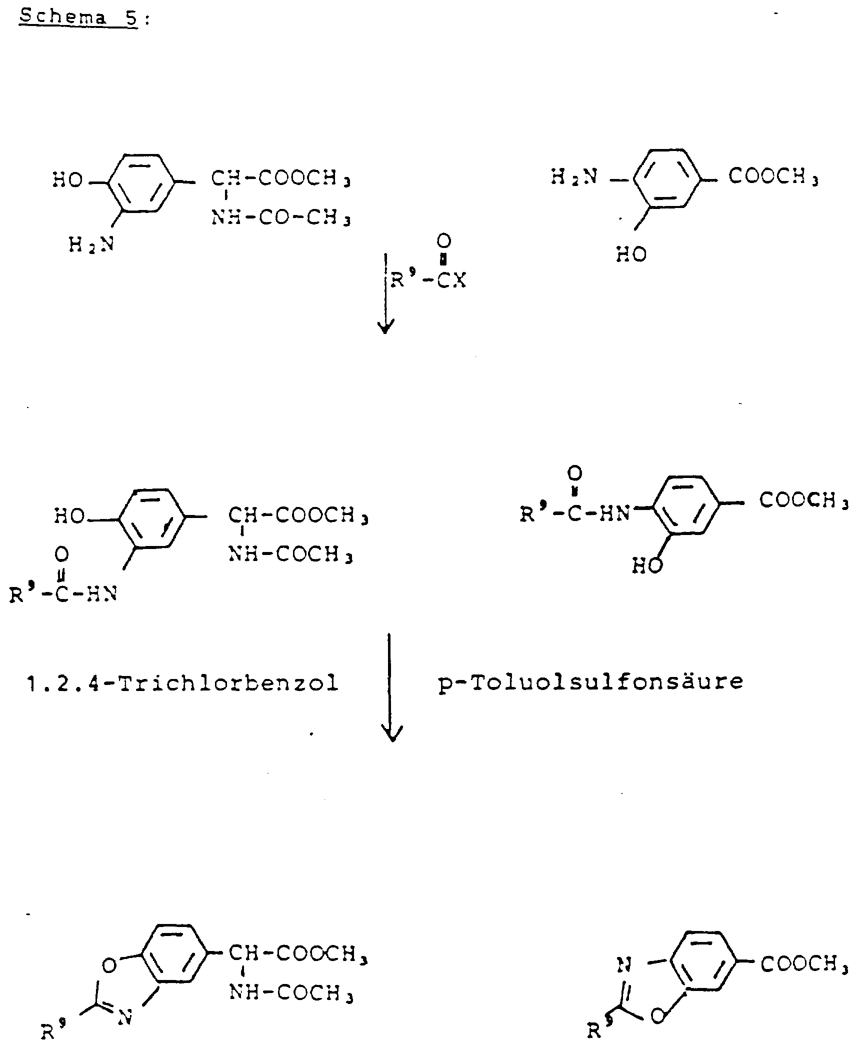 Literatur: J.W. Cornforth, Benzoxazoles and Related Systems, Heterocyclic Compounds, Vol. 5, 418, J. Wiley & Sons (1957); R. Lakham et al., Advances in Oxazole Chemistry, Advances in Heterocyclic Chemistry, Vol. 17, 99, Academic Press (1974).

### 4) Benztriazolyl- und Benzthiadiazolyl-glycine

Für die Synthese von substituierten Benztriazolyl- und Benzthiadiazolylglycinen verwendet man 3.4-Diaminobenzoesäure und p-Aminobenzoesäureethylester als Ausgangsmaterial. Die neuen benzokondensierten Carbonsäuren bzw. -ester werden beispielsweise nach folgendem Syntheseschema hergestellt: 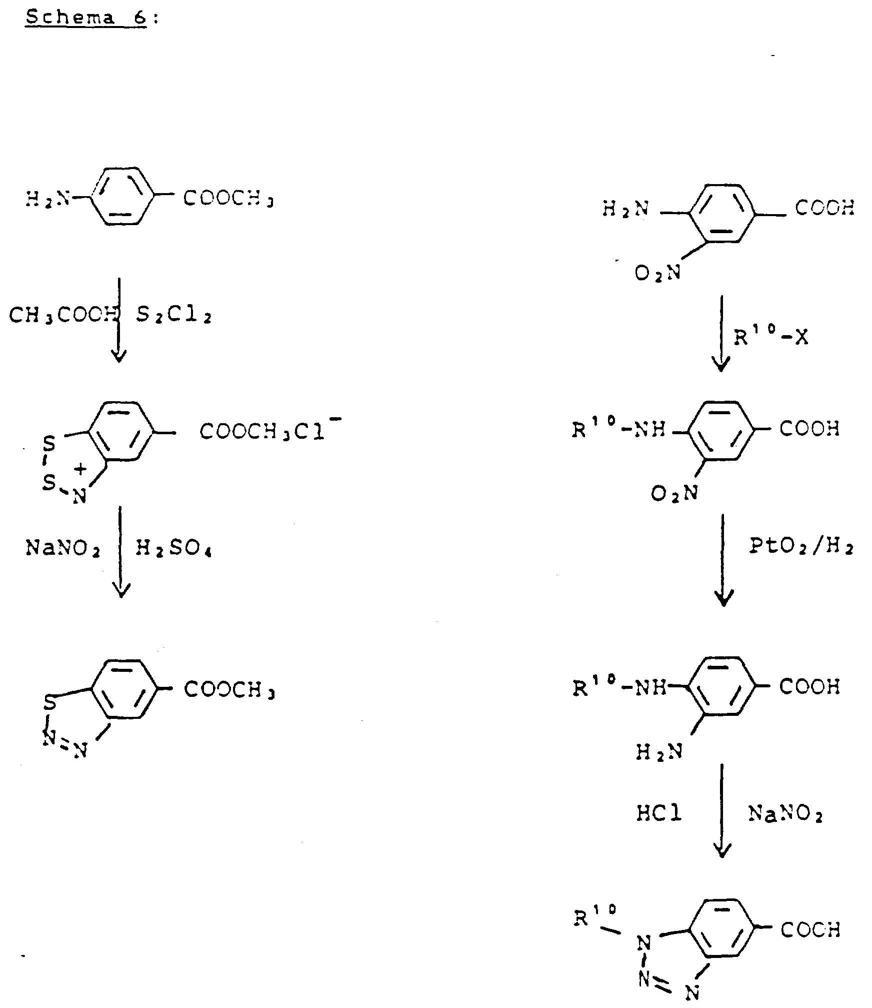 Literatur: J.B. Carr, J. Heterocyclic Chem. 9, 1149 (1972)

Die folgenden Cephalosporin-Grundkörper der Formel (II) werden zur Herstellung der erfindungsgemäßen Verbindungen der Formel (I) eingesetzt.
(R³, R⁴, R⁵ haben die oben angegebene Bedeutung).

### 1) Cephalosporin-Grundkörper (IIIa)

Die verwendeten Cephalosporin-Grundkörper, Derivate von 7-Amino-3-methyl-3-cephem-4-carbonsäure (7-ACCA), 7-Amino-3-chlor-3-cephem-4-carbonsäure (7-ACCA), 7-Amino-3-methoxy-3-cephem-4-carbonsäure, die in J. Med. Chem. 12, 310 (C.W. Ryan et al., 1969), US-Patent 3 994 884 und Deutsche Offenlegungsschrift 26 06 196 beschrieben sind, werden durch folgende Formeln dargestellt:

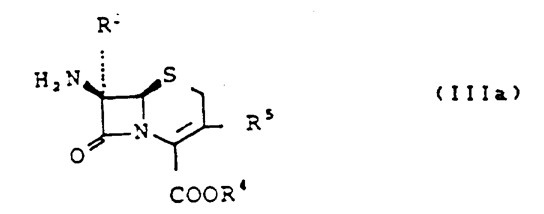

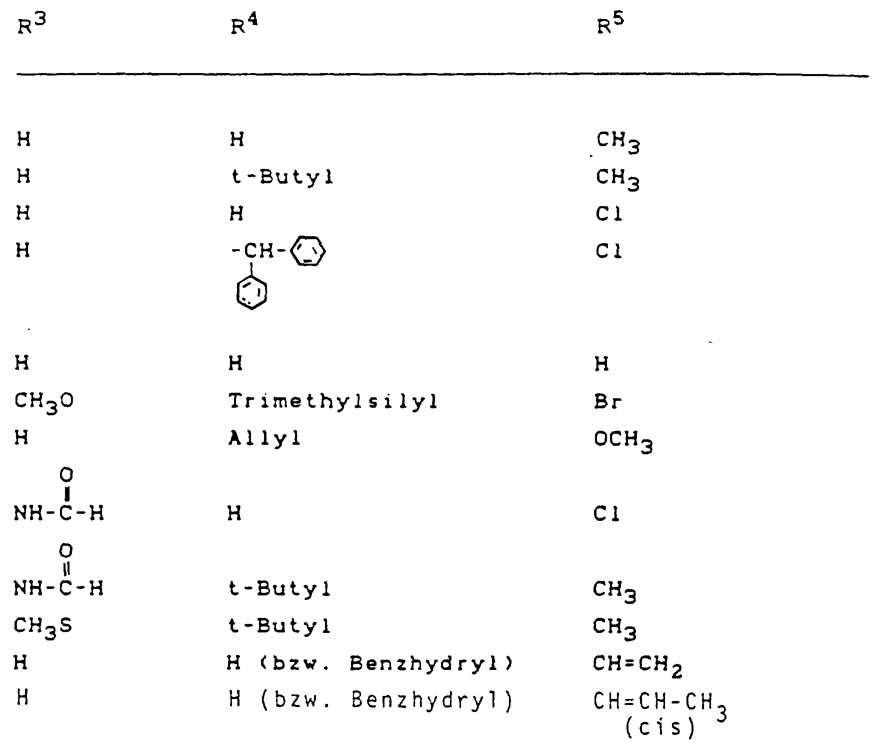

Ganz besonders bevorzugte erfindungsgemäße Verbindungen der Formel I sind in den Herstellungsbeispielen und in den folgenden Tabellen angeführt:

### a) Cephalosporine (Tab. 1, 2)

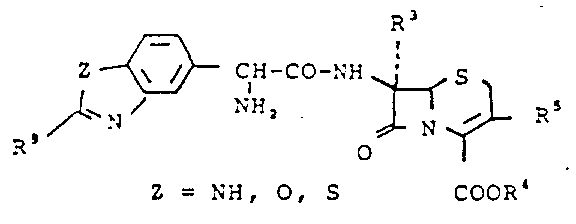

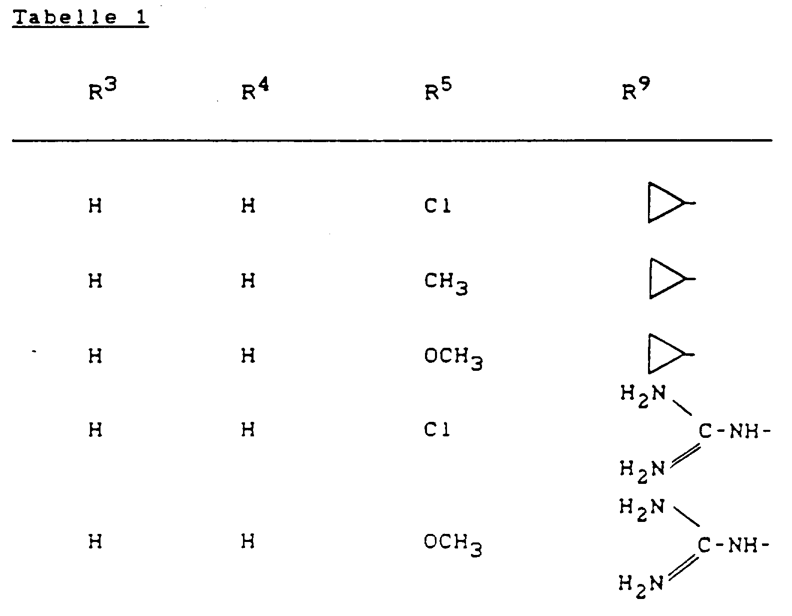

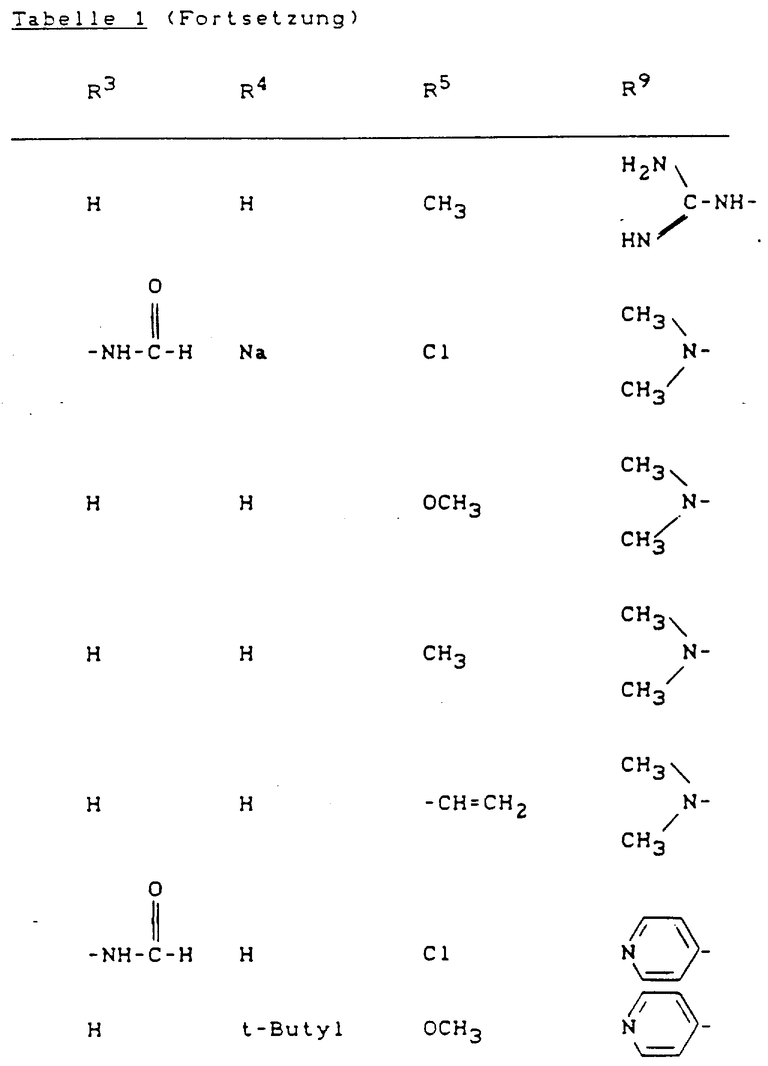

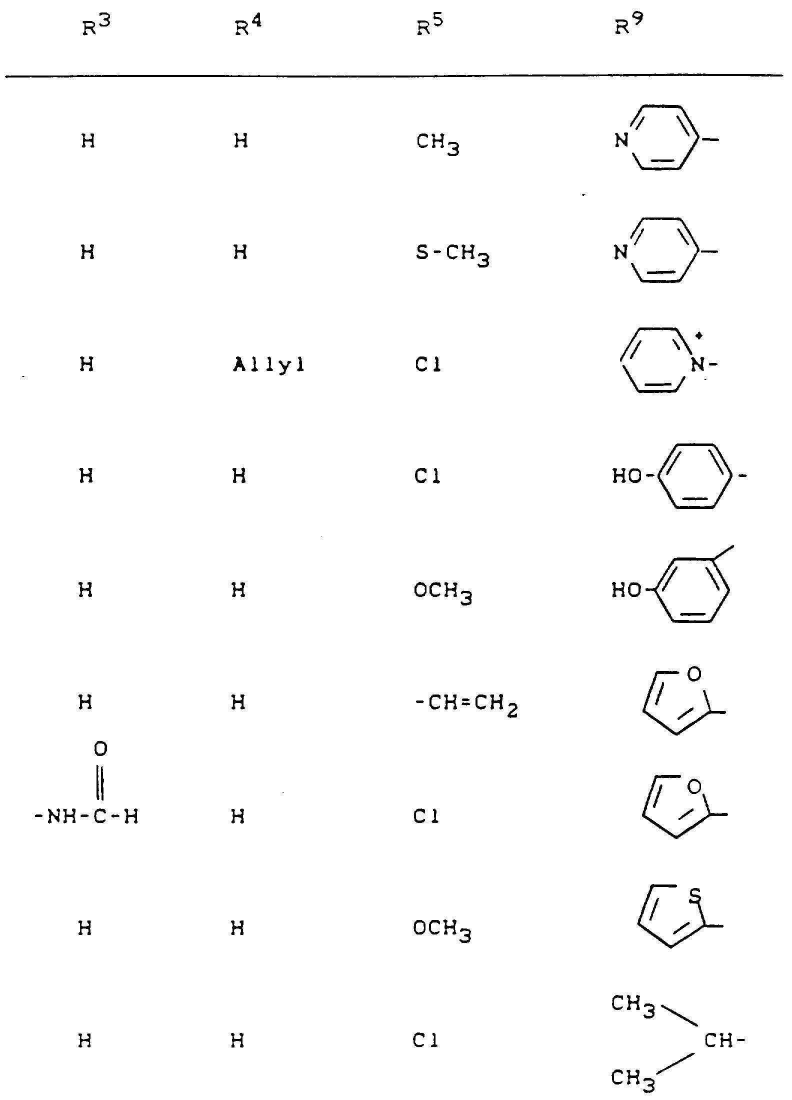

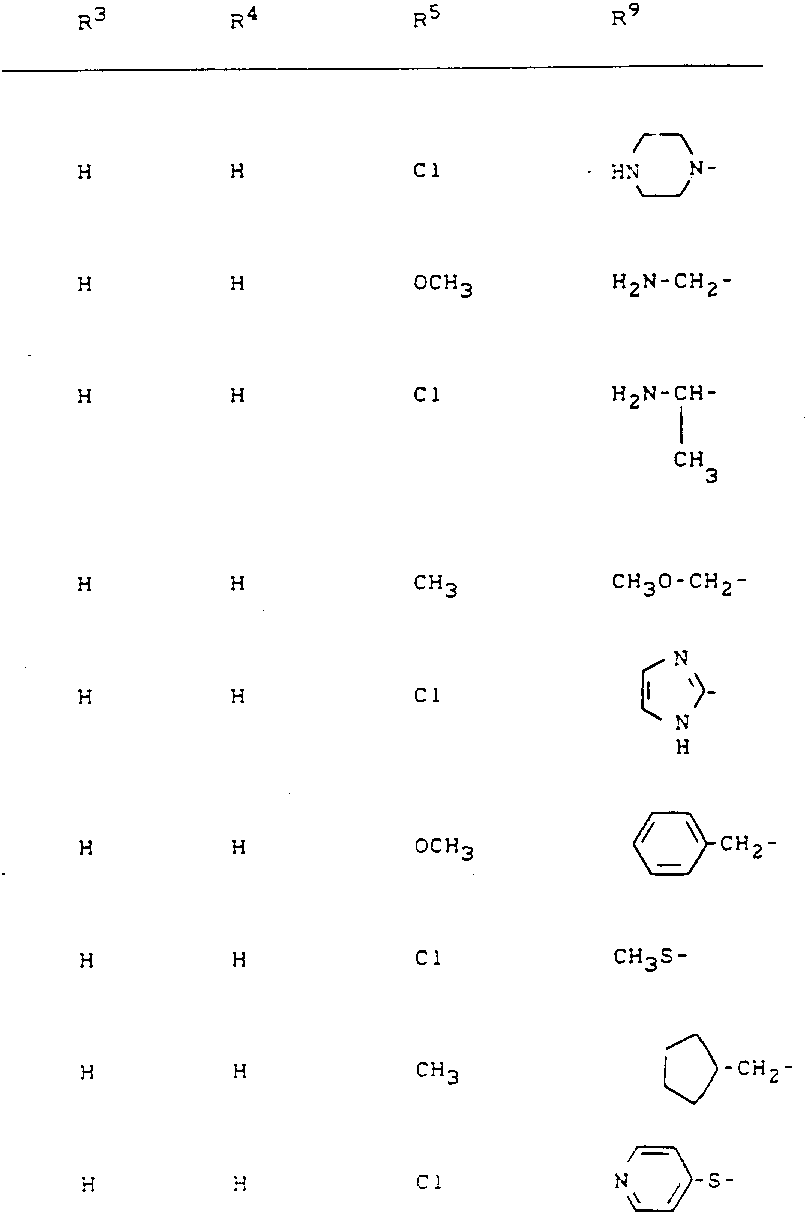

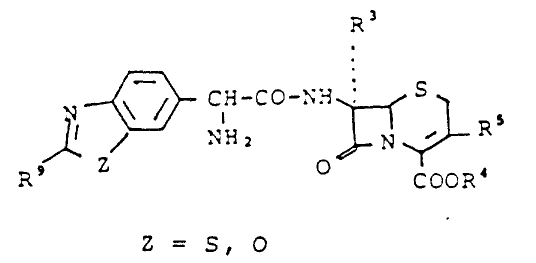

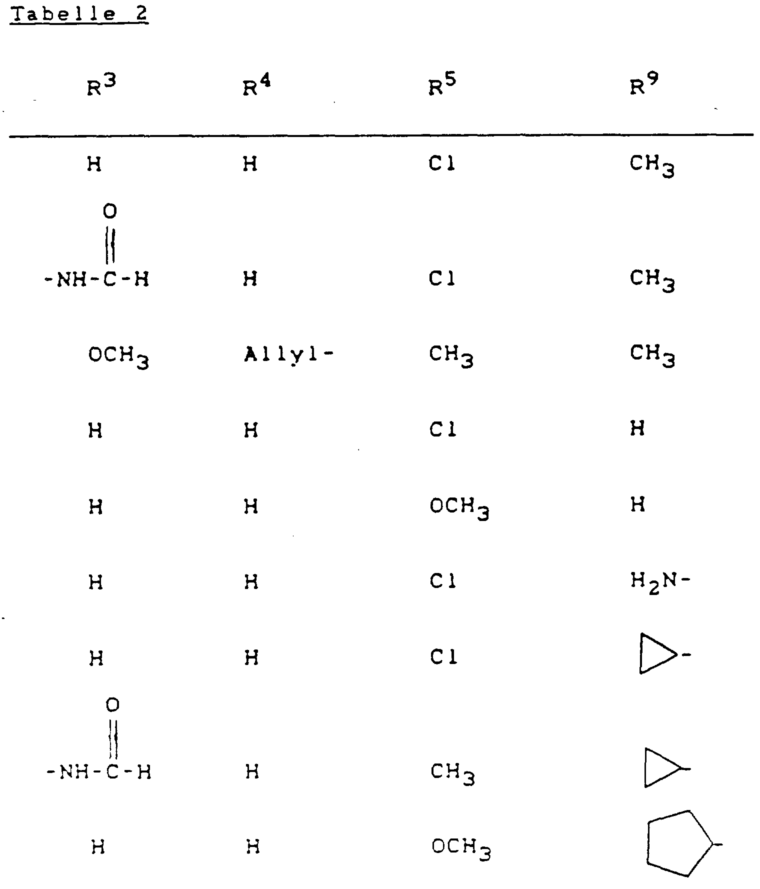

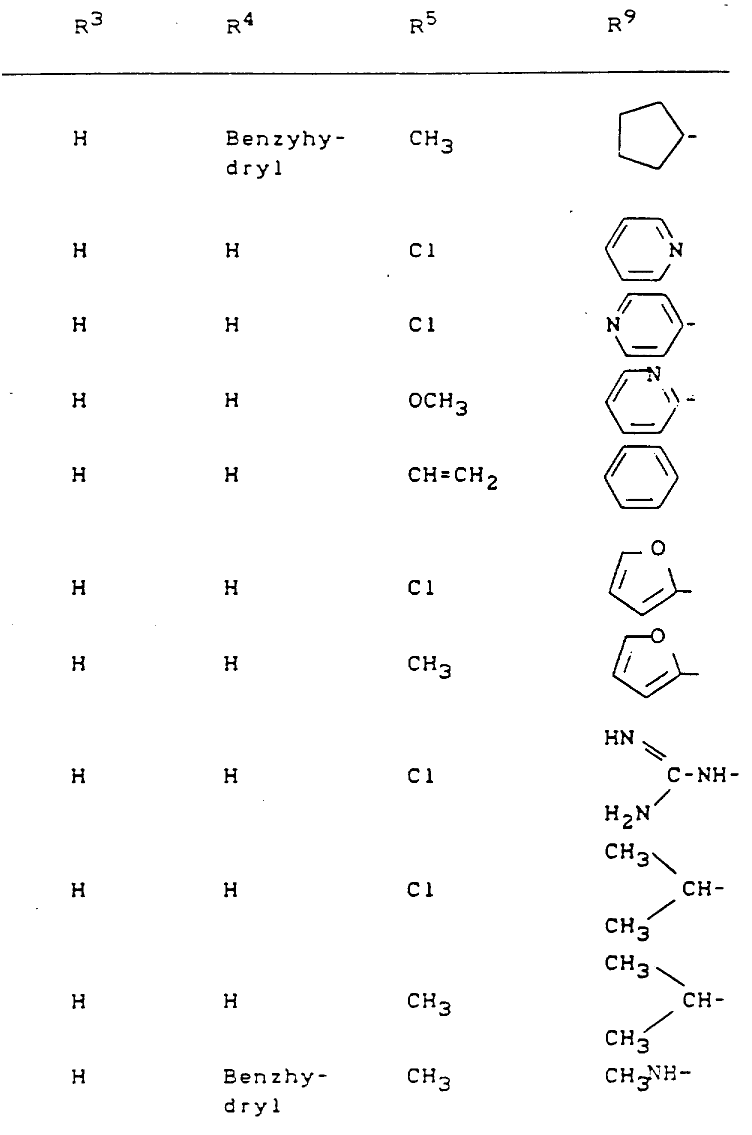

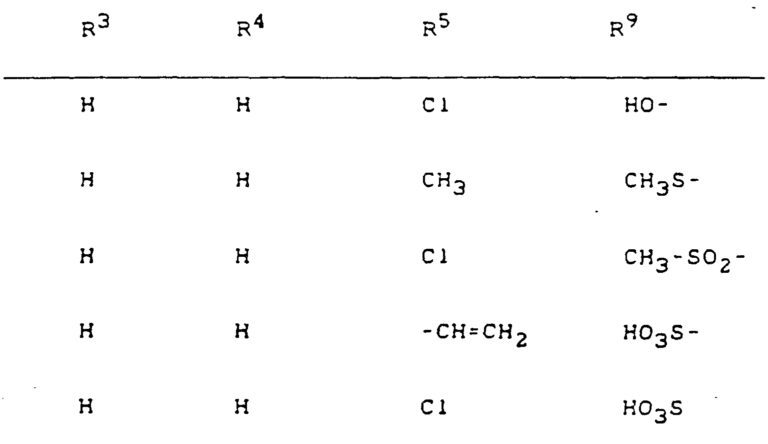

Die erfindungsgemäßen Verbindungen der Formel I weisen bei geringer Toxizität ein breites antibakterielles Spektrum gegen gram-positive und gram-negative Keime auf, insbesondere gegen Staphylokokken, Streptokokken, Enterokokken und Haemophilus influenzae.

Bei parenteraler oder insbesondere oraler Verabreichung sind die neuen Verbindungen gegen Mikroorganismen wie Staphylokokken, z.B. Staph. aureus, Staph. epidermidis; Streptokokken wie z.B. Streptococcus pyogenes, Streptococcus faecalis, Enterobakteriaceen, Escherichia coli, Klebsiella, Salmonella, Shigella, Proteus, z.B. Proteus mirabilis, sehr gut wirksam.

Diese Eigenschaften ermöglichen ihre Verwendung als chemotherapeutische Wirkstoffe in der Human- und Tiermedizin.

In der folgenden Tabelle sind die minimalen Hemmkonzentrationen (MHK-Werte, µg/ml) für die dort aufgeführten erfindungsgemäßen Verbindungen der Formel I im Vergleich mit Cefaclor [M. Gorman et al., Cefaclor, Chronicles of Drug Discovery, Vol. 2, 49, J. Wiley & Sons (1983)] angegeben. Die MHK-Werte werden durch den Agarverdünnungstest mit Hilfe eines Multipoint-Inokulators bestimmt, wobei die Ablesung nach 18 bis 24-stündiger Bebrütung bei 37°C erfolgt. Als Wuchsmedium wird Isosensitest-Agar verwendet.

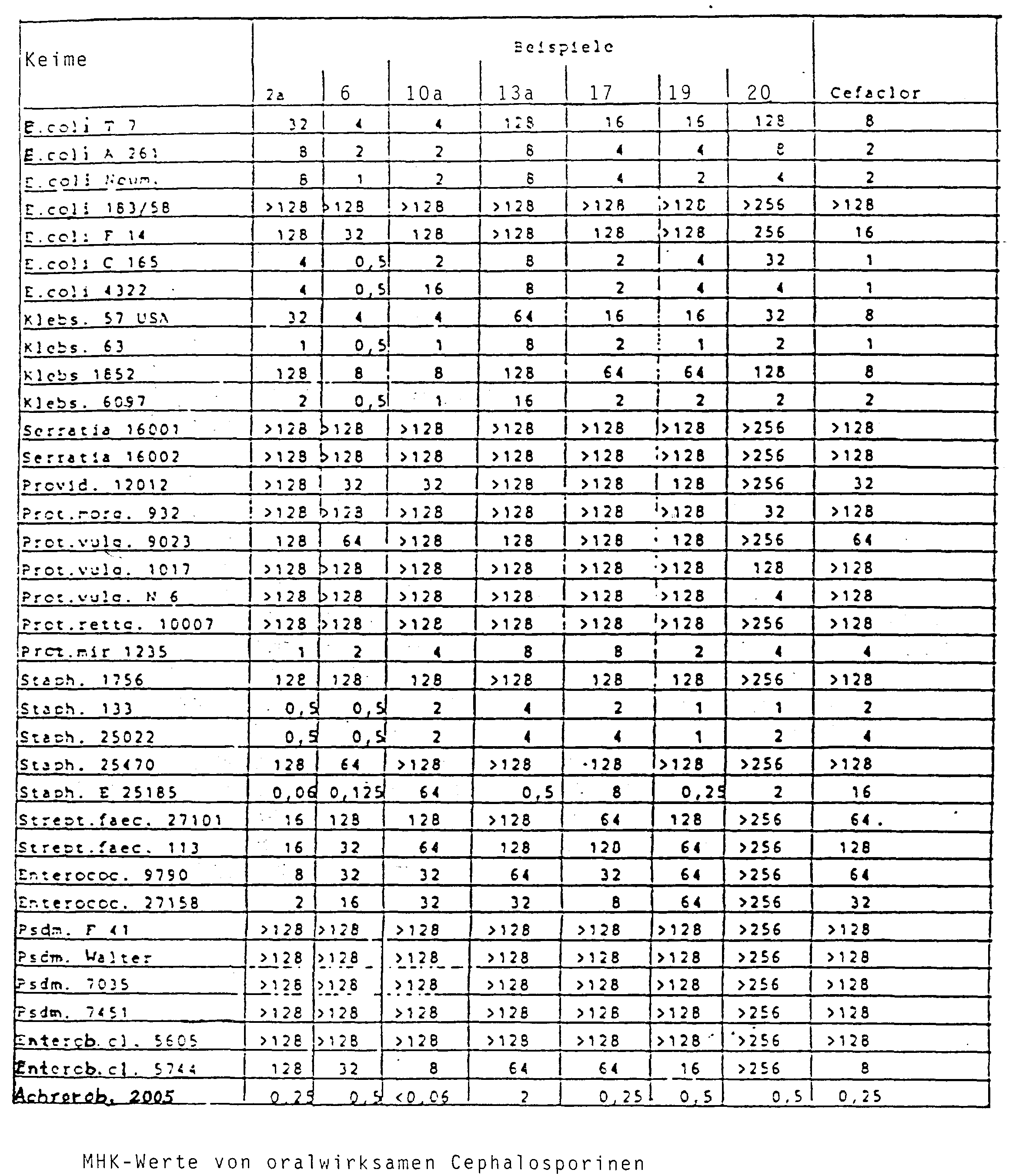

Beispielsweise können lokale und/oder systemische Erkrankungen behandelt und/oder verhindert werden, die durch die oben genannten Erreger oder durch deren Mischungen verursacht werden. Als Krankheiten, die durch die erfindungsgemäßen Verbindungen verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffen enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffen enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiummmhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Losungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,4-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenos oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis 1000, vorzugsweise 10 bis 200 mg/kg Körpergewicht je 24 Stunden, gegebenenfalls in Form mehrerer Einzelgaben, zur Erzielung der gewünschten Ergebnissee zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Korpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resorbierbarkeit aus.

Die erfindungsgemäßen Verbindungen können zum Zwecke der Erweiterung des Wirkungsspektrums und um eine Wirkungssteigerung zu erreichen auch mit anderen antimikrobiell aktiven Verbindungen und Lactamaseinhibitoren, z.B. mit Clavulansäure und Penicillinen, die besonders penicillinase-resistent sind, oder mit Aminoglykosidantibiotika, wie z.B. Aminoglykosidantibiotica, wie z.B. Gentamicin, Sisomicin, Kanamicin, Amikacin oder Tobramicin kombiniert werden.

Die Erfindung wird anhand der folgenden Beispiele weiter belegt:

### Beispiel 1

DL-7-(2-Aminobenzothiazol-6-ylglycylamido)-3-chlor-3-cephem-4-carbonsaure 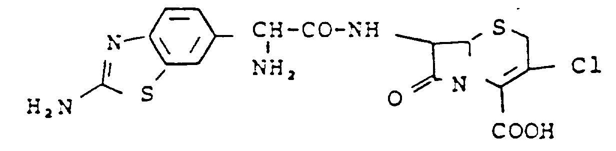
a) 2-Amino-6-hydroxymethyl-benzothiazol (1a)
50 g (0,225 mol) 2-Amino-benzothiazol-carbonsäure-6-ethylester werden in 1000 ml THF suspendiert, auf -70°C gekühlt und 562,5 ml (0,675 mol) Diisobutylaluminiumhydrid (DIBAL, 20 %ig in Toluol, 1,2 molar) unter Stickstoff langsam zugetropft. Die Reaktionslösung wird über Nacht bei -70°C bis -40°C und anschließend noch 3 Std. ohne Kühlung gerührt. Danach kühlt man auf -70°C, tropft 61,5 ml Wasser hinzu (stark exotherme Reaktion) und läßt auf Raumtemperatur kommen. Man gibt 305 ml gesättigte Kochsalzlosung hinzu und rührt 1 Stunde bei 20°C. Ausgefallenes Aluminiumhydroxid wird abgesaugt, mit THF nachgewaschen und das Filtrat bis zur Trockene eingeengt (36 g). Der Rückstand wird in siedendem Ethanol gelöst, von etwas Unlöslichem abfiltriert und das Filtrat bis zur Trockne eingedampft.
Ausbeute: 25,5 g (63 %)
C₈H₈N₂OS (180,2)
- NMR (DMSO):: δ = 4,5 (d, 2H), 5,13 (t, 1H), 7,18 (d, 1H), 7,3 (d, 1H), 7,4 (s, 2H), 7,59 (s, 1H) ppm.

b) 2-Aminobenzothiazol-6-carboxaldehyd (1b)
110,5 g (0,613 mol) 1a werden in 200 ml THF mit 319,8 g (3,678 mol) Mangan (IV)-oxid 3 Tage bei Raumtemperatur gerührt. Die Reaktionsmischung wird filtriert, der Filterrückstand mit THF gewaschen und das Filtrat i.Vak. eingeengt.
Ausbeute: 74 g (68 %)
C₈H₆N₂OS (178,1)
- NMR (DMSO):: δ = 7,48 (d, J=7,5 Hz, 1H), 7,8 (d, J=7,5 Hz, 1H), 8.08 (s, 2H), 8,26 (s, 1H), 9,92 (s, 1H) ppm.

c) 5-(2-Aminobenzothiazol-6-yl)-2,4-imidazolidindion (1c)
22,5 g (0,126 mol) 1b gelöst in 400 ml Methanol werden zu einer Lösung von 50,9 g (0,531 mol) Ammoniumcarbonat und 9,6 g (0,196 mol) Natriumcyanid in 400 ml Wasser zugetropft und 20 Std. bei 60°C gerührt. Nach Abdestillieren des Methanols i.Vak. wird die Restlösung bei 0°C mit 2 N HCl auf pH 2 angesäuert, danach mit 2 N Natronlauge auf pH 4 zurückgestellt und das ausgefallene Produkt abgesaugt.
Ausbeute: 20,5 g (65 %)

| C₁₀H₈N₄O₂S (248,3) | | | | |
|---|---|---|---|---|
| Ber.: | C 48,38 | H 3,25 | N 22,56 | S 12,91 |
| Gef.: | C 48,4 | H 3,5 | N 21,2 | S 12,3 |

- NMR (DMSO):: δ = 5,17 (s, 1H), 7,17 (q, 1H), 7,35 (d, 1H), 7,57 (s, 1H), 7,62 (s, 1H), 8,4 (s, 1H), 10,6-10,82 breites s, 1H) ppm.

d) DL-α-Amino-α-(2-aminobenzothiazol-6-yl)essigsäure (DL-6-Amino-benzothiazolylglycin, 1d)
20 g (0,081 mol) 1c werden mit 19,4g(0,81 mol) Lithiumhydroxid in 400 ml Wasser unter Rühren 24 Stdn. auf 100°C erhitzt. Die Lösung wird heiß filtriert, der Filterrückstand mit heißem Wasser nachgewaschen und das Filtrat bei Eiskühlung mit konz. HCl auf pH 2 angesäuert. Man rührt 15 Minuten bei 0°C und stellt die Lösung mit dem ausgefallenen Produkt auf pH 4,5 zurück. Der Niederschlag wird abgesaugt, mit Wasser/Aceton nachgewaschen und i.Vak. getrocknet.
Ausbeute: 10,4 g (56 %)

| C₉H₉N₃O₂S · 1/2 H₂O (232,3) | | | | |
|---|---|---|---|---|
| Ber.: | C 45,05 | H 4,20 | N 17,51 | S 13,36 |
| Gef.: | C 45,3 | H 4,6 | N 15,5 | S 13,4 |

NMR (DCOOD): δ = 5,11 (s, 1H), 7,8 (s, 2H) 8,11 (s, 1H) ppm.
e) DL-α-t-Butyloxycarbonylamino-α-(2-t-butyloxycarbonylaminobenzothiazol-6-yl)essigsäure (di-Boc-Derivat) und DL-α-t-Butyloxycarbonylamino-α-(2-aminobenzothiazol-6-yl)essigsäure (Mono-Boc-Derivat) (1e).
20 g (0,0896 mol) 1d werden in 100 ml Wasser, 130 ml Dioxan suspendiert und mit 140 ml 2 N Natronlauge in Lösung gebracht. Anschließend werden 78,1 g (0,358 mol) Di-t-butyldicarbonat in 30 Minuten zugetropft und über Nacht gerührt. Dioxan wird abdestilliert, die Restlösung mit H₂O verdünnt und mit Essigester/Petrolether gewaschen. Die wäßrige Phase wird bei Eiskühlung mit 2N HCl auf pH 2 angesäuert und zweimal mit Essigester/THF (1:1) extrahiert. Nach Waschen mit Kochsalzlösung, Trocknen über Na₂SO₄ und Einengen der organischen Phase erhält man ein Gemisch von Mono- und Di-Boc-Derivat.
Ausbeute: 24,7 g
Die Chromatographie erfolgt an Kieselgel (Merck, 0,04-0,063 mm) mit Laufmitteln in folgender Reihenfolge:
1. 2000 ml CH₂Cl₂ 2. 4000 ml CH₂Cl₂/Methanol (10:0,5) 3. CH₂Cl₂/Methanol (10:1) 4. CH₂Cl₂/Methanol (1:1),
Di-Boc-Derivat (1e):
Ausbeute: 8,7 g (26 %)
C₁₉H₂₅N₃O₆S (423,5)
- NMR (DMSO):: δ = 1,37 (s, 9H), 1,52 (s, 9H), 5,12 (d, 1H), 7,45 (d, 2H), 7,65 (d, J=7,5 Hz, 1H), 7,96 (s, 1H) ppm.
Mono-Boc-Derivat:
Ausbeute: 9,2 g (32 %)
C₁₄H₁₇N₃O₄S (323,4)
NMR (DMSO): δ = 1,36 (s, 9H), 4,98 (d, 1H), 7,2-7,3 (q, 2H), 7,48 (s, 2H), 7,54 (s, 1H) ppm.
f) DL-7-[2-(t-Butyloxycarbonylamino)-2-(2-t-butyloxycarbonylaminobenzothiazol-6-yl)acetamido]-3-chlor-3-cephem-4-carbonsäure (1f)

### Aktivierung der Precursorsäure:

5,1 g (12 mmol) 1e werden in 40 ml DMF gelöst, mit 1,69 ml (12 mmol) Triethylamin versetzt, bei -40°C 1,48 ml (12 mmol) Pivaloylchlorid tropfenweise zugespritzt und 3 Std. bei -30°C bis -15°C gerührt.

### Zubereitung der Aminkomponente:

2,83 g (12 mmol) 7-Amino-3-chlor-3-cephem-4-carbonsaure (7-ACCA) werden in 20 ml THF und 10 ml Wasser suspendiert und mit konz. Triethylamin in Lösung gebracht (pH 8,3). Danach werden 5 ml DMF zugegeben, um eine Phase zu bekommen.

### Kupplung und Isolierung:

Die 7-ACCA-Lösung wird zum gebildeten Anhydrid bei -40°C zugespritzt und im Kältebad gerührt. Nach 1 Std. gibt man 10-20 ml H₂O hinzu und stellt den pH mit 10 % Triethylamin/THF auf 7,3 ein. Nach weiteren 2 Std. gibt man 200 ml H₂O hinzu, versetzt unter Rühren mit Essigester/THF (2:1) und säuert bei 0°C auf pH 1,7 an. Die organische Schicht wird abgetrennt, mit Kochsalzlösung gewaschen, getrocknet, bis auf 30 ml eingeengt, in Petrolether eingerührt, ausgefallenes Produkt abgesaugt und getrocknet.
Ausbeute: 6,2 g (81 %), Gehalt: 66 % nach HPLC.
C₂₆H₃₀ClN₅O₈S₂ (640,1)
- NMR (DMSO):: δ = 1,39 (s, 9H), 1,52 (s, 9H), 3,5-4,02 (breites m, 2H), 5,1-5,24 (dd, 1H), 5,38 (d, 1H), 5,62-5,8 (dd, 1H), 7,42-7,53 (m, 1H), 7,68 (d, 1H), 7,98 (s, 1H) ppm.

g) DL-7-(2-Aminobenzothiazol-6-ylglycylamido)-3-chlor-3-cephem-4-carbonsäure (1g)

8,4 g (13,1 mmol) 1f werden in 50 ml Methylenchlorid gelöst, bei 0°C 0,5 ml Anisol und 50 ml Trifluoressigsäure (TFE) zugegeben und 15 Minuten ohne Kühlung gerührt. Anschließend wird das Trifluoressigsäure-Methylenchlorid-Gemisch i.Vak. abdestilliert und der ölige Rückstand mit Ether versetzt. Das Trifluoracetat wird abgesaugt, mit Ether gewaschen und getrocknet.
Ausbeute: 6,5 g (89 %)
C₁₆H₁₄ClN₅O₄S₂·CF₃COOH (553,9)
HPLC-Gehalt: (Hibar 250-4, RP 8, 10 µm, 254 nm, 3 ml/min, Laufmittel: 1000 ml H₂O-40 ml
Acetonitril-1ml TFE)
Retention: 1,10 (1d, 10,8 %), 3,08 (L-Form, 46,5 %), 3,82 (D-Form, 38,5 %).

Analog werden nach den vorstehend offenbarten Methoden u.a. folgende Verbindungen hergestellt (Beispiele 2 - 21):
2. D-7-(2-Aminobenzothiazol-6-ylglycylamido)-3-chlor-3-cephem-4-carbonsäure und L-Form;
3. DL-7-(2-Aminobenzothiazol-6-ylglycylamido)-3-methyl-3-cephem-4-carbonsäure 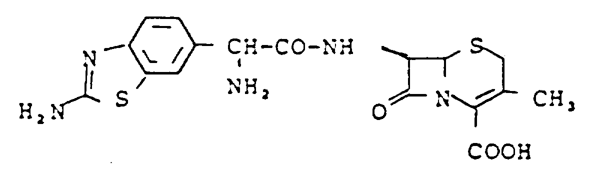
4. D-7-(2-Aminobenzothiazol-6-ylglycylamido)-3-methyl-3-cephem-4-carbonsäure und L-Form;
5. DL-7-(2-Amino-6-methylbenzothiazol-4-ylglycylamido)-3-chlor-3-cephem-4-carbonsäure 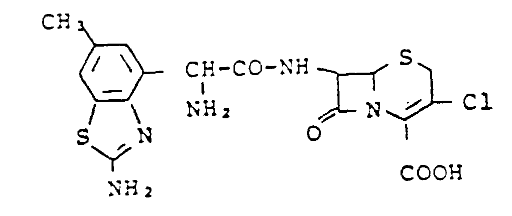
6. DL-7-(Benzimidazol-5 (6)-ylglycylamido)-3-chlor-3-cephem-4-carbonsäure 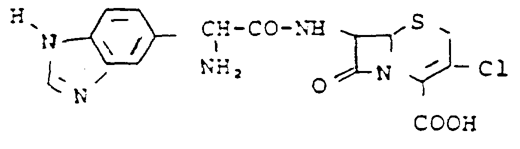
7. D-7-(Benzimidazol-5(6)-ylglycylamido)-3-chlor-3-cephem-4-carbonsäure und L-Form;
8. DL-7-(Benzimidazol-5(6)-ylglycylamido)-3-methyl-3-cephem-4-carbonsäure 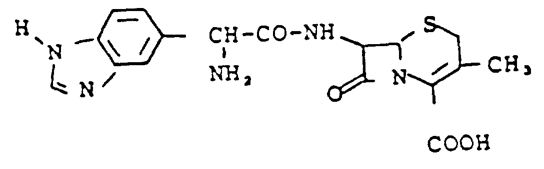
9. DL-7-(2-Amino-1H-benzimidazol-5(6)-ylglycylamido)-3-chlor-3-cephem-4-carbonsäure 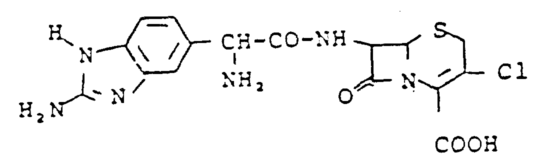
10. D-7-(2-Amino-1H-benzimidazol-5-ylglycylamido)-3-chlor-3-cephem-4-carbonsäure und L-Form;
11. DL-7-(2-Amino-1H-benzimidazol-5-ylglycylamido)-3-methyl-3-cephem-4-carbonsäure 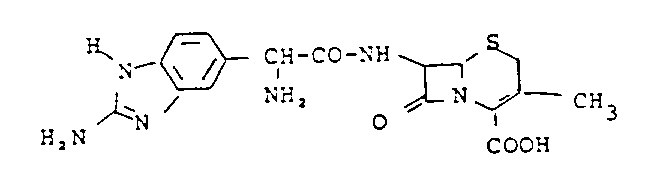
12. DL-7-(2-Trifluormethyl-1H-benzimidazol-5-ylglycylamido)-3-chlor-3-cephem-4-carbonsäure 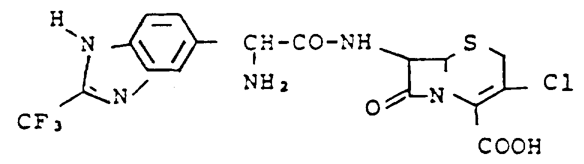
13. D-7-(2-Trifluormethyl-1H-benzimidazol-5-ylglycylamido)-3-chlor-3-cephem-4-carbonsäure und L-Form;
14. DL-7-(2-Methyl-1H-benzimidazol-5-ylglycylamido)-3-chlor-3-cephem-4-carbonsäure 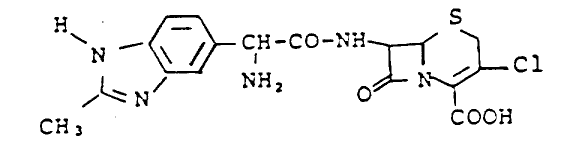
15. D-7-(2-Methyl-1H-benzimidazol-5-ylglycylamido)-3-chlor-3-cephem-4-carbonsäure und L-Form;
16. DL-7-(2,3-Dihydro-2-oxo-1H-benzimidazol-5-ylglycylamido)-3-chlor-3-cephem-3-carbonsäure 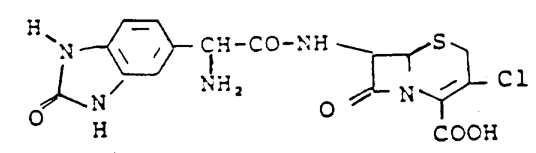
17. D-7-(2,3-Dihydro-2-oxo-1H-benzimidazol-5-ylglycylamido)-3-chlor-3-cephem-4-carbonsäure und L-Form;
18. D-7-(2-Aminobenzoxazol-5-ylglycylamido)-3-chlor-3-cephem-4-carbonsäure 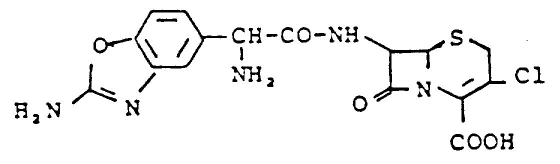
19. DL-7-(Benztriazol-5(6)-ylglycylamido)-3-chlor-3-cephem-4-carbonsäure 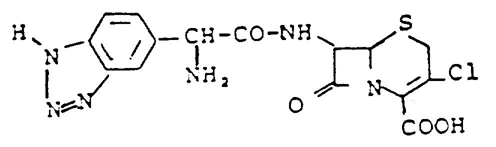
20. D-7-(2-Aminobenzothiazol-5-yl-glycylamido)-3-chlor-3-cephem-4-carbonsäure

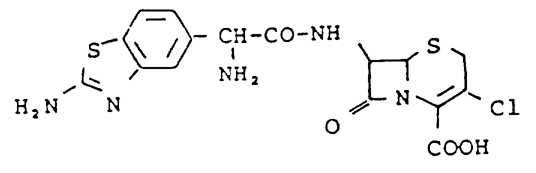

## Patentansprüche (Patentansprüche für folgende(n) Vertragsstaat(en): BE, CH, DE, FR, GB, IT, LI, NL, SE)

1. β-Lactamverbindungen der allgemeinen Formel (I) 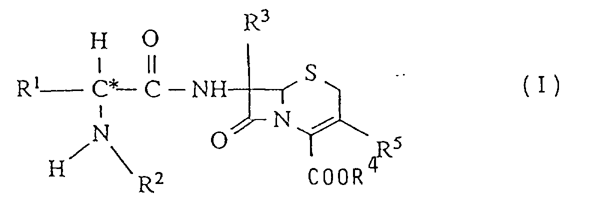 worin
R⁵
- für Wasserstoff,
- für Fluor, Chlor, Brom,
- für geradkettiges oder verzweigtes, gesättigtes oder ungesättigtes Alkyl mit bis zu 5 C-Atomen steht, das gegebenenfalls substituiert ist durch ein oder mehrere Fluor, Chlor, Brom, Alkoxy, Alkylthio mit jeweils 1 bis 3 C-Atomen Carbamoyloxy, Acetyloxy, Benzoyloxy, oder durch einen Rest der Formel 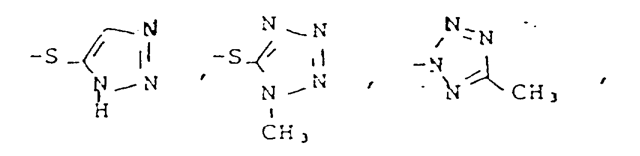 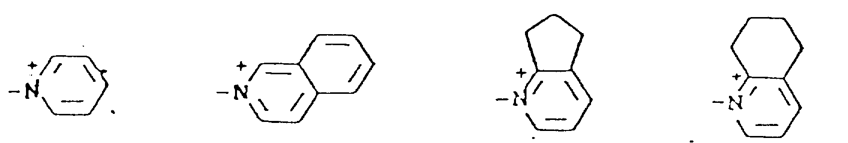 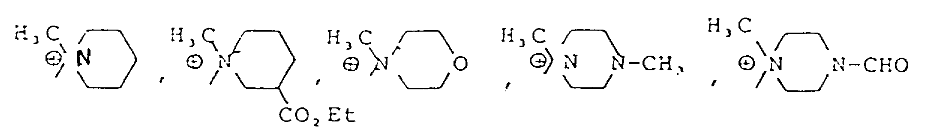 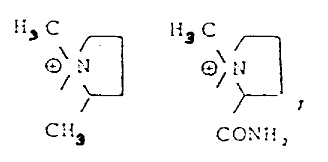 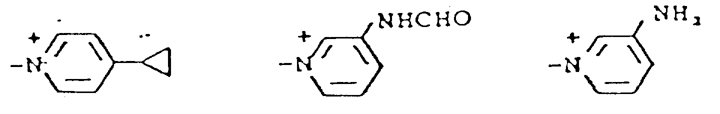 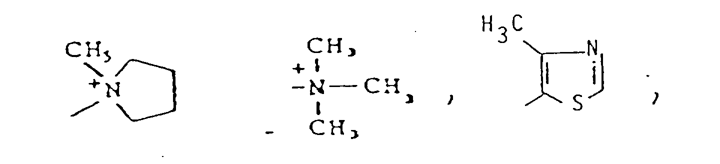
R¹ für einen Rest der Formel 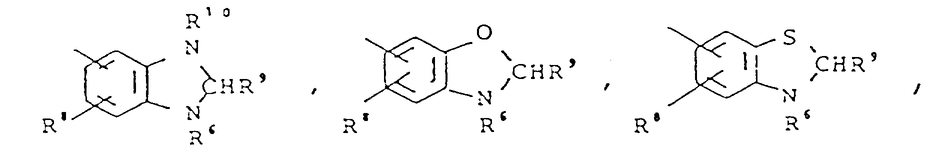 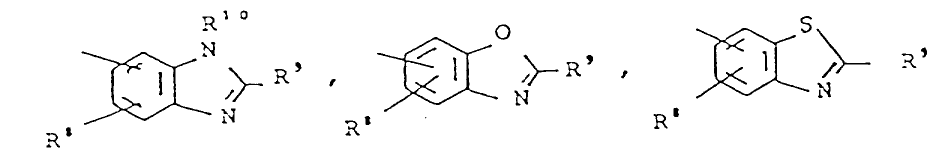 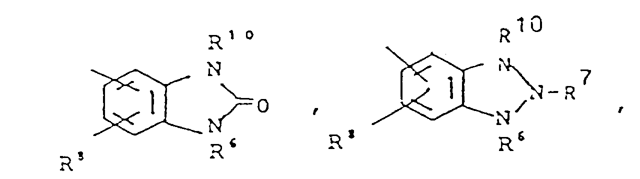 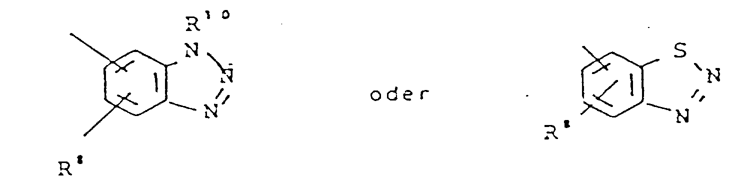
steht,
wobei
R⁶
- für Wasserstoff,
- für Hydroxy oder Amino steht, oder
- für geradkettiges, verzweigtes oder cyclisches,
gesättigtes oder ungesättigtes, gegebenenfalls durch ein oder mehrere Fluor, Chlor, Brom, Amino, Hydroxy oder Phenyl substituiertes Alkyl mit bis zu 8 C-Atomen steht, oder
- für Phenyl steht,
R⁷
- für Wasserstoff, oder
- für geradkettiges, verzweigtes oder cyclisches, gesättigtes oder ungesättigtes Alkyl mit bis zu 8 C-Atomen steht, das gegebenenfalls substituiert ist durch ein oder mehrere Fluor, Chlor, Brom, C₁-C₄-Alkoxy, Hydroxy, Carboxy, Phenyl, SO₃H oder eine Aminogruppe, oder
- für Phenyl steht,
R⁸
- für Wasserstoff,
- für Alkyl, Alkoxy, Alkylthio mit jeweils 1 bis 6 C-Atomen,
- für Trifluormethyl, Trifluormethoxy,
- für Hydroxy, Mercapto, Nitro, Cyano,
- für Fluor, Chlor, Brom oder
- für eine Aminogruppe steht,
und wobei
R⁹
- die gleiche Bedeutung hat wie R⁷ und außerdem
- für Fluor, Chlor, Brom,
- für C₁-C₆-Alkoxy, C₁-C₆-Alkylthio steht,
- für eine Aminogruppe,
- für -SO₂-C₁-C₆-Alkyl, -PO(OH)₂,
- für -SO₃H oder -SO₂NH₂ steht,
- für SH, OH, S-Phenyl oder O-Phenyl,
- für Guanidino, -NHNH₂, NHOH, oder
- für ein gegebenenfalls ein- bis zweifach gleich oder verschieden durch Alkyl, Alkylthio, Alkoxy mit jeweils 1 bis 2 C-Atomen,
Fluor, Chlor, Brom, Nitro, Cyano, Hydroxy, Amino, Trifluormethyl, Trifluormethoxy oder Trifluormethylthio substituiertes Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxalyl, Chinazolyl, Piperidinyl, Oxazolyl, Thiazolyl, Isoxazolyl, Thiadiazolyl, Triazolyl oder Tetrazolyl steht,
R¹⁰
- die gleiche Bedeutung hat wie R⁶, jedoch nicht mit R⁷ eine Doppelbindung vervollständigt,
oder
R⁹ und R¹⁰ gemeinsam für eine gegebenenfalls durch Schwefel unterbrochene C₂-C₄-Methylenkette stehen,
R
- für Wasserstoff oder
- für eine leicht abspaltbare Aminoschutzgruppe steht,
R³
- für Wasserstoff,
- für Alkoxy, Alkylthio mit jeweils 1 bis 3 C-Atomen,
- für eine Aminogruppe oder
- für NHCHO steht,
und
R⁴
- für Wasserstoff,
- für eine in der β-Lactamchemie übliche Carboxyschutzgruppe,
- für -CH₂-O-CO-C(CH₃)₃,
- für -CH(CH₃)-O-CO-O-C₂H₅, -CH₂-O-CO-CH₃,
- für den Rest der Formel 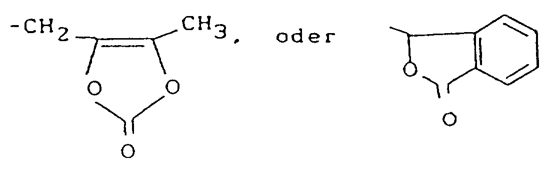 oder
- für Na⁺, Li⁺, K⁺ oder NH₄ ⁺ steht.

2. β-Lactamverbindungen nach Anspruch 1, dadurch gekennzeichnet, daß, wenn R für eine Aminoschutzgruppe steht, R für eine leicht abspaltbare Aminoschutzgruppe aus der Gruppe bestehend aus tert.- Butoxycarbonyl (Boc), Trityl (Trt), Benzyloxycarbonyl (Z), Formyl, Chloracetyl oder 1-Methyl-2-ethoxycarbonyl-vinyl steht, oder daß, wenn R⁴ eine Carboxyschutzgruppe ist, R⁴ für eine in der β-Lactamchemie übliche Schutzgruppe aus der Gruppe bestehend aus tert.-Butyl, Decyl, 2,2,2-Trichlorethyl, Benzyl, 4-Methoxybenzyl, 4-Nitrobenzyl, Tri- bzw. Diphenylmethyl, Acetoxymethyl, Allyl oder Trimethylsilyl steht.

3. Verfahren zur Herstellung von β-Lactamverbindungen der allgemeinen Formel (I) gemäß den Ansprüchen 1 und 2, dadurch gekennzeichnet, daß man Verbindungen der allgemeinen Formel II 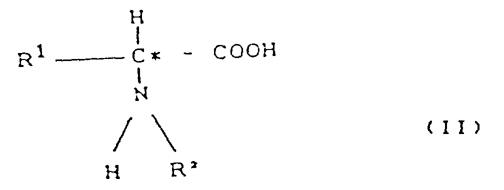 in welcher
R¹ die in den Ansprüchen 1 und 2 angegebene Bedeutung hat und in welcher
R für eine Aminoschutzgruppe steht,
nach Aktivierung der Carboxylgruppe durch Überführung in ein gemischtes Anhydrid, nach Überführung in das Mesylat oder nach Überführung in einen aktivierten Ester mit Verbindungen der
allgemeinen Formel (III) 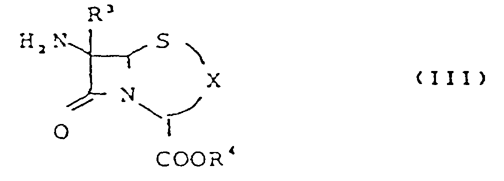 in welcher
R₃, R₄ und X die in den Ansprüchen 1 und 2 angegebenen Bedeutung haben, zur Umsetzung bringt, dann gegebenenfalls Schutzgruppen abspaltet und die gewünschten Salze oder aus Salzen die freien Säuren herstellt.

4. β-Lactamverbindungen der allgemeinen Formel (I) gemäß den Ansprüchen 1 und 2,
und deren D-, L- und D,L-Formen zur Anwendung bei der therapeutischen Behandlung des menschlichen oder tierischen Körpers.

5. Verwendung von β-Lactamverbindungen der allgemeinen Formel (I) gemäß den Ansprüchen 1 und 2,
und deren D-, L- und D,L-Formen für die Herstellung von Arzneimitteln.

6. Arzneimittel, enthaltend β-Lactamverbindungen der allgemeinen Formel (I) gemäß den Ansprüchen 1 und 2, und deren D-, L- und D,L-Formen.

7. Tierfuttermittel bzw. Tierfuttermittel-Prämixe, gekennzeichnet durch einen Gehalt an Cephalosporinen gemäß den Ansprüchen 1 und 2.

## Patentansprüche (Patentansprüche für folgende(n) Vertragsstaat(en): AT)

1. Verfahren zur Herstellung von β-Lactamverbindungen der allgemeinen Formel (I) 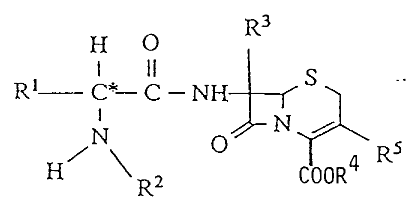 worin
R⁵
- für Wasserstoff,
- für Fluor, Chlor, Brom,
- für geradkettiges oder verzweigtes, gesättigtes oder ungesättigtes Alkyl mit bis zu 5 C-Atomen steht, das gegebenenfalls substituiert ist durch ein oder mehrere Fluor, Chlor, Brom. Alkoxy, Alkylthio mit jeweils 1 bis 3 C-Atomen Carbamoyloxy, Acetyloxy, Benzoyloxy, oder durch einen Rest der Formel 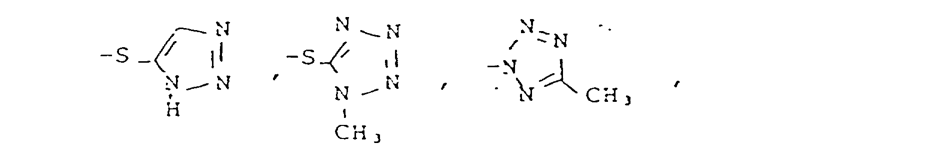 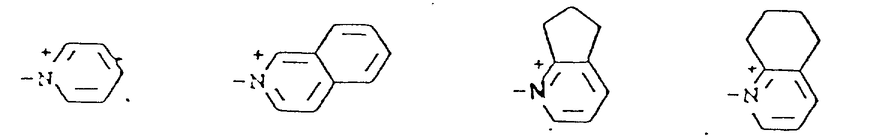 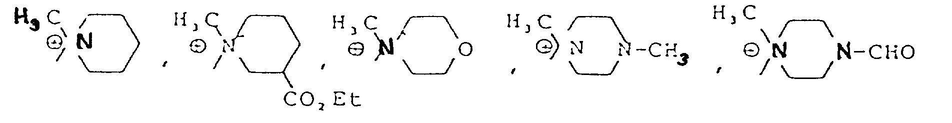 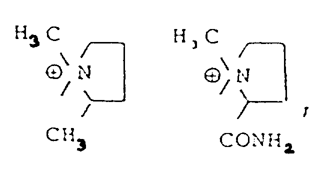 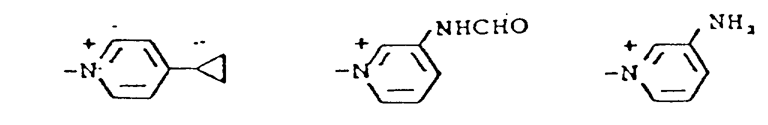 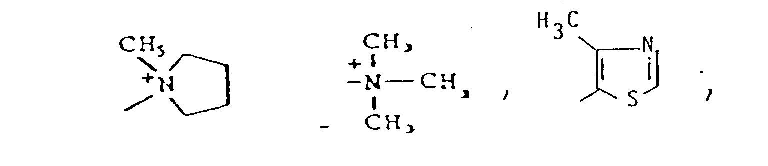
R¹ für einen Rest der Formel 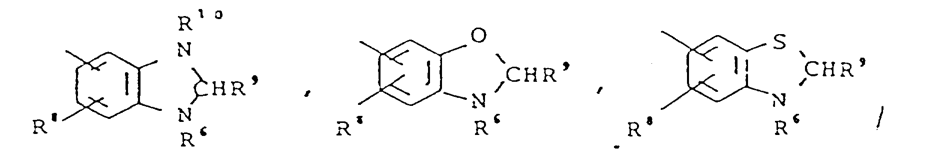 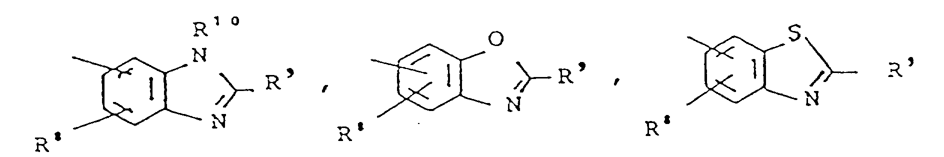 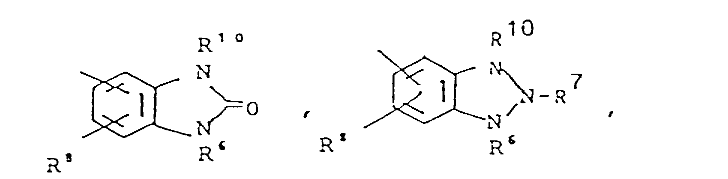 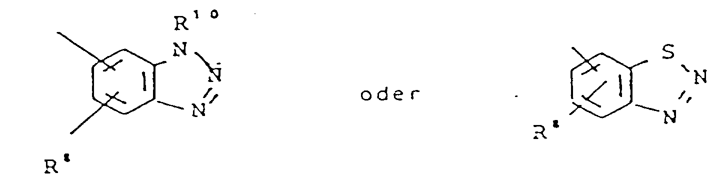
steht,
wobei
R⁶
- für Wasserstoff,
- für Hydroxy oder Amino steht, oder
- für geradkettiges, verzweigtes oder cyclisches,
gesättigtes oder ungesättigtes, gegebenenfalls durch ein oder mehrere Fluor, Chlor, Brom, Amino, Hydroxy oder Phenyl substituiertes Alkyl mit bis zu 8 C-Atomen steht, oder
- für Phenyl steht,
R⁷
- für Wasserstoff, oder
- für geradkettiges, verzweigtes oder cyclisches, gesättigtes oder ungesättigtes Alkyl mit bis zu 8 C-Atomen steht, das gegebenenfalls substituiert ist durch ein oder mehrere Fluor, Chlor, Brom, C₁-C₄-Alkoxy, Hydroxy, Carboxy, Phenyl, SO₃H oder eine Aminogruppe, oder
- für Phenyl steht,
R⁸
- für Wasserstoff,
- für Alkyl, Alkoxy, Alkylthio mit jeweils 1 bis 6 C-Atomen,
- für Trifluormethyl, Trifluormethoxy,
- für Hydroxy, Mercapto, Nitro, Cyano,
- für Fluor, Chlor, Brom oder
- für eine Aminogruppe steht,
und wobei
R⁹
- die gleiche Bedeutung hat wie R⁷ und außerdem
- für Fluor, Chlor, Brom,
- für C₁-C₆-Alkoxy, C₁-C₆-Alkylthio steht,
- für eine Aminogruppe,
- für -SO₂-C₁-C₆-Alkyl, -PO(OH)₂,
- für -SO₃H oder -SO₂NH₂ steht,
- für SH, OH, S-Phenyl oder O-Phenyl,
- für Guanidino, -NHNH₂, NHOH, oder
- für ein gegebenenfalls ein- bis zweifach gleich oder verschieden durch Alkyl, Alkylthio, Alkoxy mit jeweils 1 bis 2 C-Atomen,
Fluor, Chlor, Brom, Nitro, Cyano, Hydroxy, Amino, Trifluormethyl, Trifluormethoxy oder Trifluormethylthio substituiertes Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxalyl, Chinazolyl, Piperidinyl, Oxazolyl, Thiazolyl, Isoxazolyl, Thiadiazolyl, Triazolyl oder Tetrazolyl steht,
R¹⁰
- die gleiche Bedeutung hat wie R⁶, jedoch nicht mit R⁷ eine Doppelbindung vervollständigt,
oder
R⁹ und R¹⁰ gemeinsam für eine gegebenenfalls durch Schwefel unterbrochene C₂-C₄-Methylenkette stehen,
R
- für Wasserstoff oder
- für eine leicht abspaltbare Aminoschutzgruppe steht,
R³
- für Wasserstoff,
- für Alkoxy, Alkylthio mit jeweils 1 bis 3 C-Atomen,
- für eine Aminogruppe oder
- für NHCHO steht,
und
R⁴
- für Wasserstoff,
- für eine in der β-Lactamchemie übliche Carboxyschutzgruppe,
- für -CH₂-O-CO-C(CH₃)₃,
- für -CH(CH₃)-O-CO-O-C₂H₅, -CH₂-O-CO-CH₃,
- für den Rest der Formel 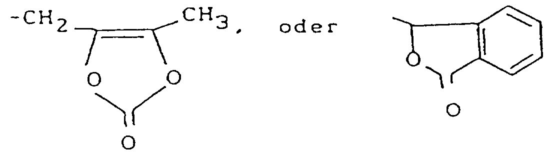 oder
- für Na⁺, Li⁺, K⁺ oder NH₄ ⁺ steht
dadurch gekennzeichnet, daß man Verbindungen der allgemeinen Formel II 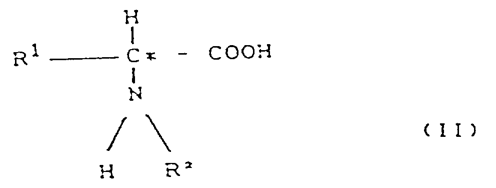 in welcher
R¹ die in den Ansprüchen 1 und 2 angegebene Bedeutung hat und in welcher
R für eine leicht abspaltbare Aminoschutzgruppe steht,
nach Aktivierung der Carboxylgruppe durch Überführung in ein gemischtes Anhydrid, nach Überführung in das Mesylat oder nach Überführung in einen aktivierten Ester mit Verbindungen der
allgemeinen Formel (III) 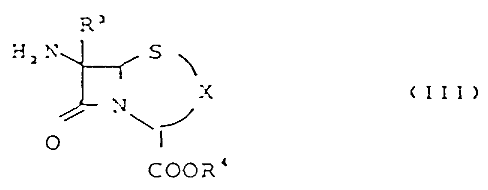 in welcher
R³, R⁴ und X die oben angegebene Bedeutung haben, zur Umsetzung bringt, dann gegebenenfalls Schutzgruppen abspaltet und die gewünschten Salze oder aus Salzen die freien Säuren herstellt.

2. Verfahren nach Anspruch 1, dadurch gekennzeichnet, daß man für die Kupplung der Aminosäuren der Formel (II) an β-Lactame der Formel (III) aus der Cephalosporin- bzw. Penicillinchemie bekannte Methoden verwendet.

3. Verfahren nach den Ansprüchen 1 und 2, dadurch gekennzeichnet, daß man Aminosäuren der allgemeinen Formel (II) aktiviert und dann mit β-Lactamen der allgemeinen Formel (III), die als Salze mit einem Amin in Lösung gebracht werden, kuppelt.

4. Verfahren nach Anspruch 1 und 3, dadurch gekennzeichnet, daß man die Aktivierung mit Pivalinsäurechlorid oder Sulfonsäurederivaten der Formel (IV) zu Anhydriden der Formel (V) nach dem Schema 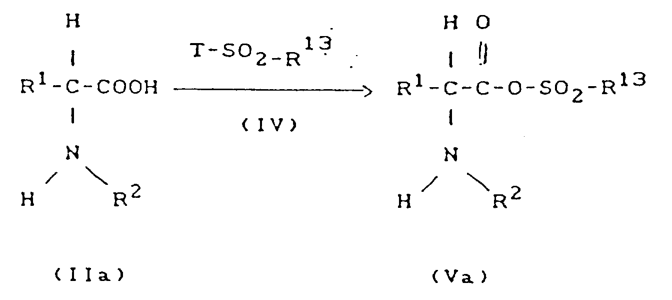 oder 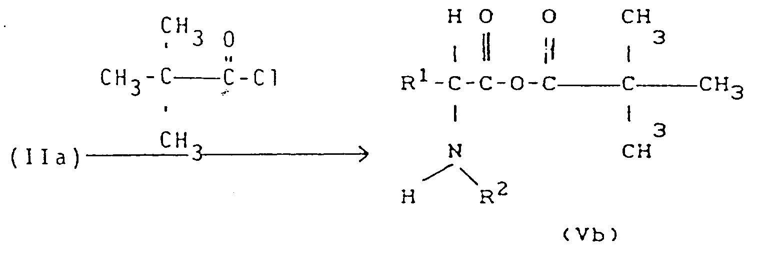 in welchen
R¹ - die in Anspruch 1 angegebene Bedeutung hat,
R - für eine leicht abspaltbare Aminoschutzgruppe steht,
T - für einen Rest R¹³-SO₂-O oder Halogen steht, und
R¹³ - für C₁-C₁₀-Alkyl steht, das gegebenenfalls substituiert ist durch Fluor, Chlor, Cyano, Phenyl, C₁-C₄-Alkyl, C₁-C₄-Alkoxy oder C₁-C₄-Alkylthio, oder
- für gegebenenfalls durch Fluor, Chlor, Brom, Cyano, C₁-C₄-Alkyl, C₁-C₄-Alkoxy, C₁-C₄-Alkylthio, C₁-C₄-Alkylcarbonyl, Nitro, Trifluormethyl substituiertes Phenyl steht,
durchführt.

5. Verfahren nach den Ansprüchen 1 bis 4, dadurch gekennzeichnet, daß man die Umsetzungen bei Temperaturen zwischen -80°C und Raumtemperatur, bevorzugt zwischen -60°C und 0°C durchführt.

6. Verwendung von β-Lactamverbindungen der allgemeinen Formel (I) gemäß den Ansprüchen 1 bis 5,
und deren D-, L- und D,L-Formen für die Herstellung von Arzneimitteln.

## Claims (Claims for the following Contracting State(s): BE, CH, DE, FR, GB, IT, LI, NL, SE)

1. β-Lactam compounds of the general formula (I) 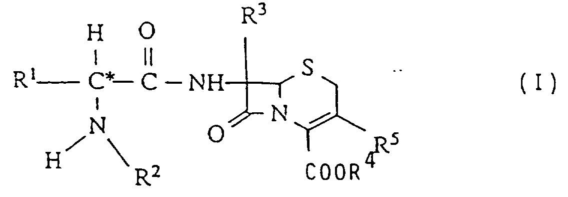 in which
R⁵
- represents hydrogen,
- represents fluorine, chlorine or bromine,
- represents straight-chain or branched, saturated or unsaturated alkyl which has up to 5 C atoms and which is optionally substituted by one or more radicals from the group consisting of fluorine, chlorine, bromine, alkoxy or alkylthio, each having 1 to 3 C atoms, carbamoyloxy, acetyloxy and benzoyloxy, or by a radical of the formula 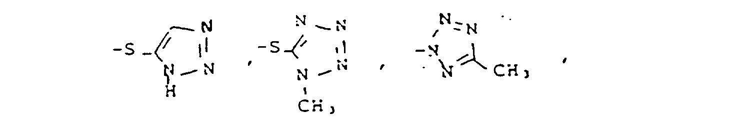 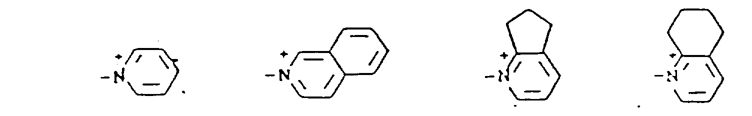 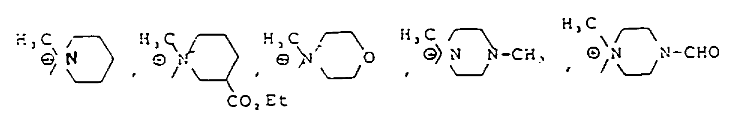 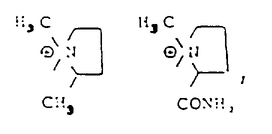 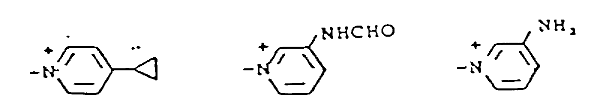 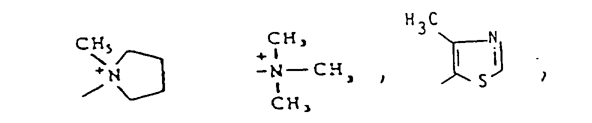
R¹ - represents a radical of the formula 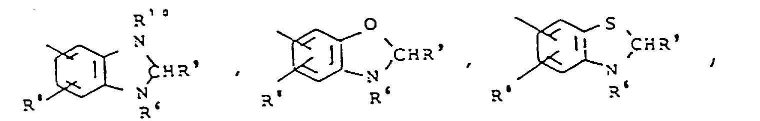 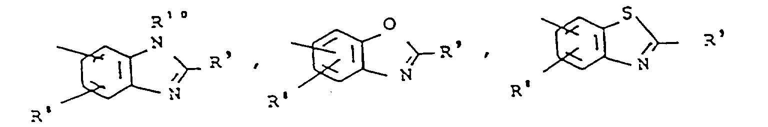 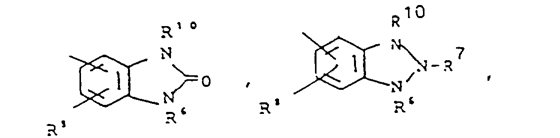 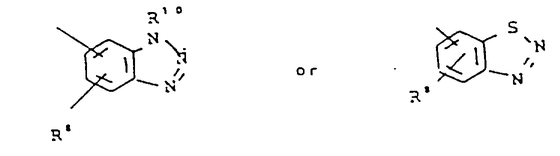
R⁶
- representing hydrogen,
- representing hydroxyl or amino, or
- representing straight-chain, branched or cyclic, saturated or unsaturated alkyl which has up to 8 C atoms and which is optionally substituted by one or more fluorine, chlorine, bromine, amino, hydroxyl or phenyl radicals, or
- representing phenyl,
R⁷
- representing hydrogen, or
- representing straight-chain, branched or cyclic, saturated or unsaturated alkyl which has up to 8 C atoms and which is optionally substituted by one or more fluorine, chlorine, bromine, C₁-C₄-alkoxy, hydroxyl, carboxyl, phenyl or SO₃H radicals or an amino group, or
- representing phenyl,
R⁸
- representing hydrogen,
- representing alkyl, alkoxy or alkylthio, each having 1 to 6 C atoms,
- representing trifluoromethyl or trifluoromethoxy,
- representing hydroxyl, mercapto, nitro or cyano,
- representing fluorine, chlorine or bromine, or
- representing an amino group,
and
R⁹
- having the same meaning as R⁷ and, additionally,
- representing fluorine, chlorine or bromine,
- representing C₁-C₆-alkoxy or C₁-C₆-alkylthio,
- representing an amino group,
- representing -SO₂-C₁-C₆-alkyl or -PO(OH)₂,
- representing -SO₃H or -SO₂NH₂,
- representing SH, OH, S-phenyl or O-phenyl,
- representing guanidino, -NHNH₂ or -NHOH, or
- representing pyrrolyl, pyrrolidinyl, pyrazolyl, imidazolyl, pyridyl, pyridazinyl, pyrimidyl, pyrazinyl, quinolyl, isoquinolyl, indolyl, quinoxalyl, quinazolyl, piperidinyl, oxazolyl, thiazolyl, isoxazolyl, thiadiazolyl, triazolyl or tetrazolyl, optionally substituted, identically or differently, once to twice, by alkyl, alkylthio or alkoxy, each having 1 to 2 C atoms, or by fluorine, chlorine, bromine, nitro, cyano, hydroxyl, amino, trifluoromethyl, trifluoromethoxy or trifluoromethylthio,
R¹⁰
- having the same meaning as R⁶ but not completing a double bond with R⁷,
or
R⁹ and R¹⁰ together representing a C₂-C₄-methylene chain which is optionally interrupted by sulphur,
R
- represents hydrogen or
- represents an amino-protective group which can readily be eliminated,
R³
- represents hydrogen,
- represents alkoxy or alkylthio, each having 1 to 3 C atoms,
- represents an amino group, or
- represents NHCHO,
and
R⁴
- represents hydrogen,
- represents a carboxyl-protective group which is customary in β-lactam chemistry,
- represents -CH₂-O-CO-C(CH₃)₃,
- represents -CH(CH₃)-O-CO-O-C₂H₅ or -CH₂-O-CO-CH₃,
- represents the radical of the formula 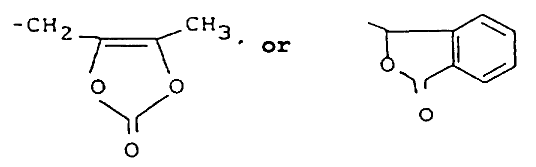 or
- represents Na⁺, Li⁺, K⁺ or NH₄ ⁺.

2. β-Lactam compounds according to Claim 1, characterised in that when R represents an amino-protective group, R represents an amino-protective group which can readily be eliminated and is of the group consisting of tert-butoxycarbonyl (Boc), trityl (Trt), benzyloxycarbonyl (Z), formyl, chloroacetyl and 1-methyl-2-ethoxycarbonyl-vinyl, or in that when R⁴ is a carboxyl-protective group, R⁴ represents a protective group which is customary in β-lactam chemistry and is of the group consisting of tert-butyl, decyl, 2,2,2-trichloroethyl, benzyl, 4-methoxybenzyl, 4-nitrobenzyl, triphenylmethyl or diphenylmethyl, acetoxymethyl, allyl and trimethylsilyl.

3. Process for the preparation of β-lactam compounds of the general formula (I) according to Claims 1 and 2, characterised in that compounds of the general formula II 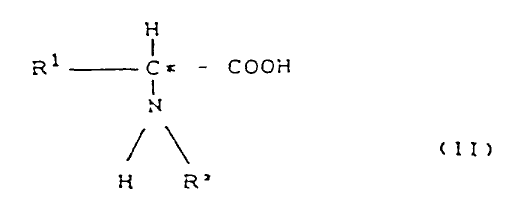 in which
R¹ has the meaning given in Claims 1 and 2 and in which
R represents an amino-protective group,
are, after activation of the carboxyl group by conversion into a mixed anhydride, after conversion into the mesylate or after conversion into an activated ester, induced to react with compounds of the general formula (III) 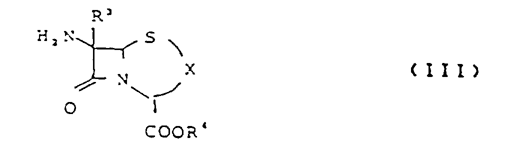 in which
R³, R⁴ and X have the meaning given in Claims 1 and 2, then, where appropriate, protective groups are eliminated and the desired salts are prepared, or the free acids are prepared from salts.

4. β-Lactam compounds of the general formula (I) according to Claims 1 and 2,
and their D-, L- and D,L-forms for use for the therapeutic treatment of the human or animal body.

5. Use of β-lactam compounds of the general formula (I) according to Claims 1 and 2,
and their D-, L- and D,L-forms for the preparation of medicaments.

6. Medicament, containing β-lactam compounds of the general formula (II) according to Claims 1 and 2, and their D-, L- and D,L-forms.

7. Animal feedstuffs or premixes for animal feedstuffs, characterised by containing cephalosporins according to Claims 1 and 2.

## Claims (Claims for the following Contracting State(s): AT)

1. Process for the preparation of β-lactam compounds of the aeneral formula (I) 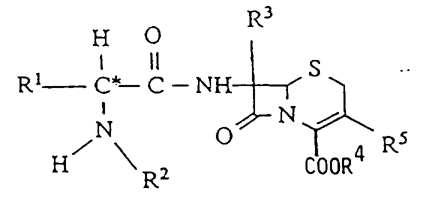 in which
R⁵
- represents hydrogen,
- represents fluorine, chlorine or bromine,
- represents straight-chain or branched, saturated or unsaturated alkyl which has up to 5 C atoms and which is optionally substituted by one or more radicals from the group consisting of fluorine, chlorine, bromine, alkoxy or alkylthio, each having 1 to 3 C atoms, carbamoyloxy, acetyloxy and benzoyloxy, or by a radical of the formula 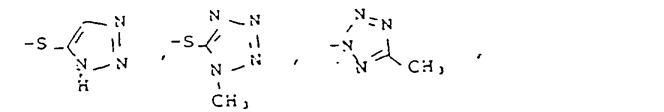 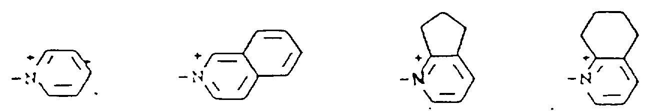 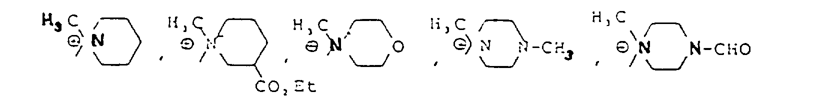 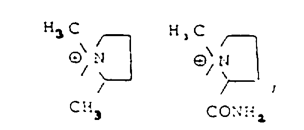 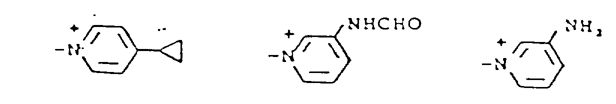 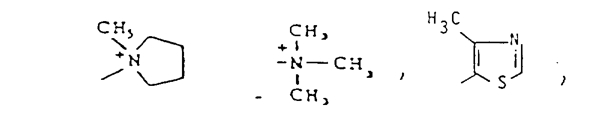
R¹ - represents a radical of the formula 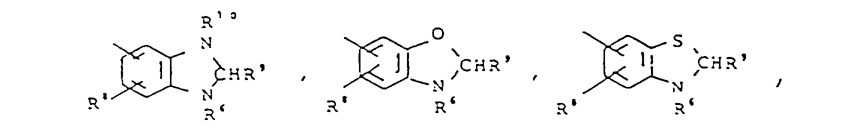 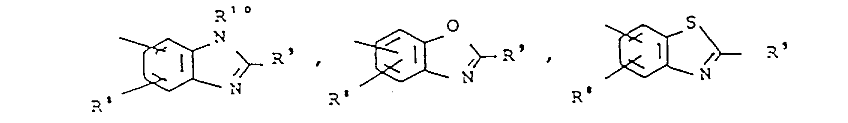 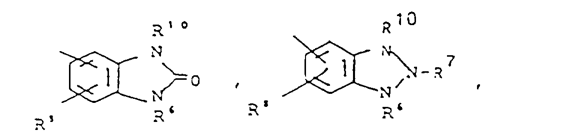 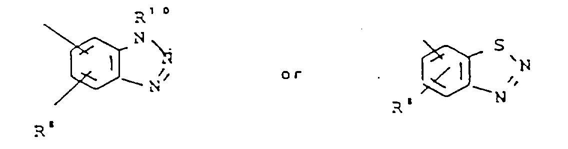
R⁶
- representing hydrogen,
- representing hydroxyl or amino, or
- representing straight-chain, branched or cyclic, saturated or unsaturated alkyl which has up to 8 C atoms and which is optionally substituted by one or more fluorine, chlorine, bromine, amino, hydroxyl or phenyl radicals, or
- representing phenyl,
R⁷
- representing hydrogen, or
- representing straight-chain, branched or cyclic, saturated or unsaturated alkyl which has up to 8 C atoms and which is optionally substituted by one or more fluorine, chlorine, bromine, C₁-C₄-alkoxy, hydroxyl, carboxyl, phenyl or SO₃H radicals or an amino group, or
- representing phenyl,
R⁸
- representing hydrogen,
- representing alkyl, alkoxy or alkylthio, each having 1 to 6 C atoms,
- representing trifluoromethyl or trifluoromethoxy,
- representing hydroxyl, mercapto, nitro or cyano,
- representing fluorine, chlorine or bromine, or
- representing an amino group,
and
R⁹
- having the same meaning as R⁷ and, additionally,
- representing fluorine, chlorine or bromine,
- representing C₁-C₆-alkoxy or C₁-C₆-alkylthio,
- representing an amino group,
- representing -SO₂-C₁-C₆-alkyl or -PO(OH)₂,
- representing -SO₃H or -SO₂NH₂,
- representing SH, OH, S-phenyl or O-phenyl,
- representing guanidino, -NHNH₂ or -NHOH, or
- representing pyrrolyl, pyrrolidinyl, pyrazolyl, imidazolyl, pyridyl, pyridazinyl, pyrimidyl, pyrazinyl, quinolyl, isoquinolyl, indolyl, quinoxalyl, quinazolyl, piperidinyl, oxazolyl, thiazolyl, isoxazolyl, thiadiazolyl, triazolyl or tetrazolyl, optionally substituted, identically or differently, once to twice, by alkyl, alkylthio or alkoxy, each having 1 to 2 C atoms, or by fluorine, chlorine, bromine, nitro, cyano, hydroxyl, amino, trifluoromethyl, trifluoromethoxy or trifluoromethylthio,
R¹⁰
- having the same meaning as R⁶ but not completing a double bond with R⁷,
or
R⁹ and R¹⁰ together representing a C₂-C₄-methylene chain which is optionally interrupted by sulphur,
R
- represents hydrogen or
- represents an amino-protective group which can readily be eliminated,
R³
- represents hydrogen,
- represents alkoxy or alkylthio, each having 1 to 3 C atoms,
- represents an amino group, or
- represents NHCHO,
and
R⁴
- represents hydrogen,
- represents a carboxyl-protective group which is customary in β-lactam chemistry,
- represents -CH₂-O-CO-C(CH₃)₃,
- represents -CH(CH₃)-O-CO-O-C₂H₅ or -CH₂-O-CO-CH₃,
- represents the radical of the formula 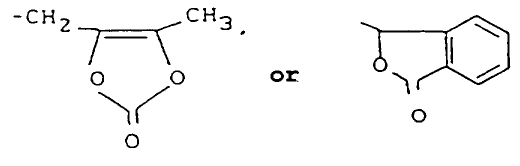 or
- represents Na⁺, Li⁺, K⁺ or NH₄ ⁺.
characterised in that compounds of the general formula II 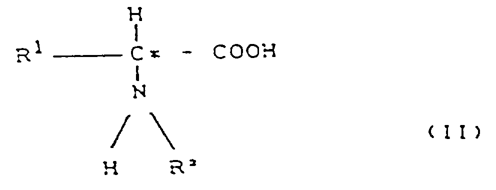 in which
R¹ has the meaning given in Claims 1 and 2 and in which
R represents an amino-protective group which can readily be eliminated,
are, after activation of the carboxyl group by conversion into a mixed anhydride, after conversion into the mesylate or after conversion into an activated ester, induced to react with compounds of the general formula (III) 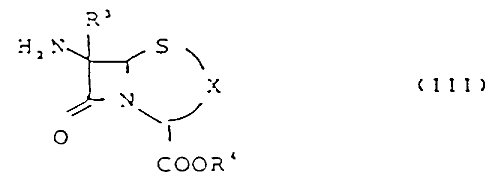 in which
R³, R⁴ and X have the abovementioned meaning, then, where appropriate, protective groups are eliminated and the desired salts are prepared, or the free acids are prepared from salts.

2. Process according to Claim 1, characterised in that methods known from cephalosporin and penicillin chemistry are used for the coupling of the amino acids of the formula (II) to β-lactams of the formula (III).

3. Process according to Claims 1 and 2, characterised in that amino acids of the general formula (II) are activated and then coupled with β-lactams of the general formula (III) which are induced to dissolve as salts with an amine.

4. Process according to Claims 1 and 3, characterised in that the activation is carried out with pivaloyl chloride or sulphonic acid derivatives of the formula (IV) to give anhydrides of the formula (V) in accordance with the scheme 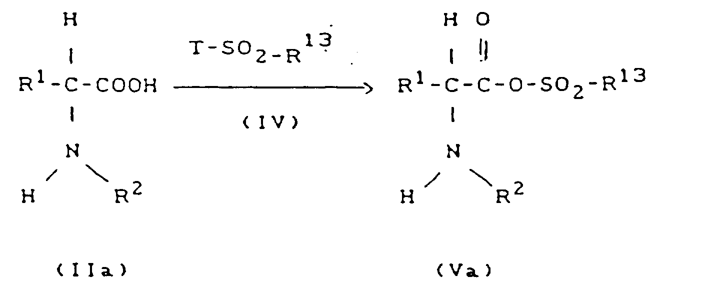 or 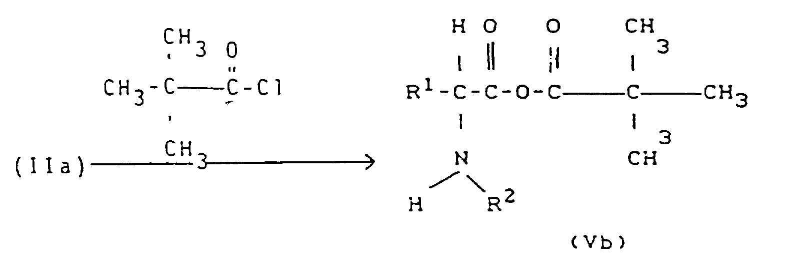 in which
R¹ - has the meaning given in Claim 1,
R - represents an amino-protective group which can readily be eliminated,
T - represents a radical R¹³-SO₂-O or halogen, and
R¹³ - represents C₁-C₁₀-alkyl which is optionally substituted by fluorine, chlorine, cyano, phenyl, C₁-C₄-alkyl, C₁-C₄-alkoxy or C₁-C₄-alkylthio, or
- represents phenyl which is optionally substituted by fluorine, chlorine, bromine, cyano, C₁-C₄-alkyl, C₁-C₄-alkoxy, C₁-C₄-alkylthio, C₁-C₄-alkylcarbonyl, nitro or trifluoromethyl.

5. Process according to Claims 1 to 4, characterised in that the reactions are carried out at temperatures between -80°C and room temperature, preferably between -60°C and 0°C.

6. Use of β-lactam compounds of the general formula (I) according to Claims 1 to 5,
and their D-, L- and D,L-forms for the preparation of medicaments.

## Revendications (Revendications pour l'(les) Etat(s) contractant(s) suivant(s): BE, CH, DE, FR, GB, IT, LI, NL, SE)

1. Composés du type de β-lactames de formule générale (I) 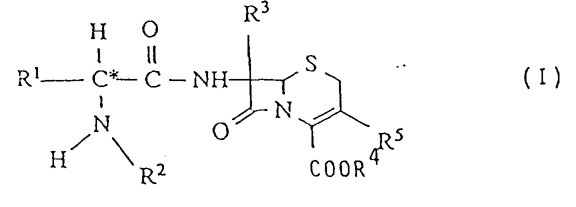 dans laquelle
R⁵ représente
- de l'hydrogène,
- du fluor, du chlore, du brome,
- un groupe alkyle linéaire ou ramifié, saturé ou non saturé, ayant jusqu'à 5 atomes de carbone, qui est substitué le cas échéant par un ou plusieurs radicaux fluoro, chloro, bromo, alkoxy, alkylthio ayant chacun 1 à 3 atomes de carbone, carbamoyloxy, acétyloxy, benzoyloxy ou par un reste de formule 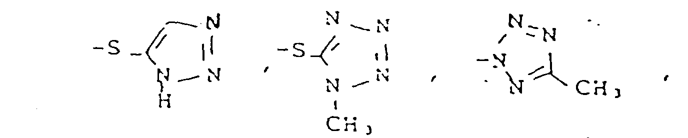 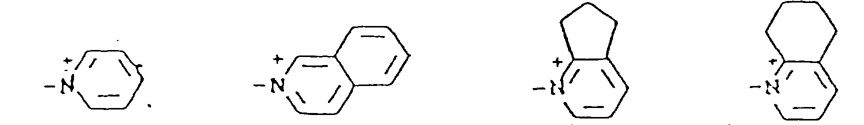 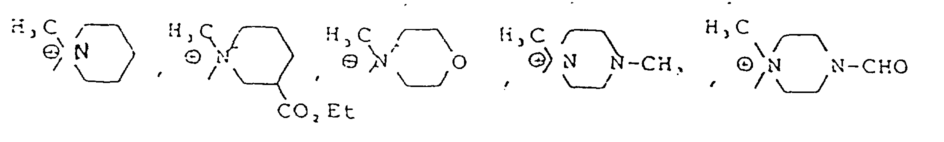 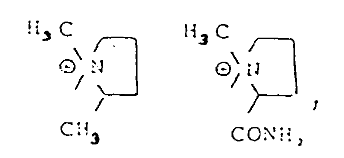 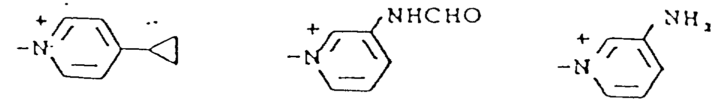 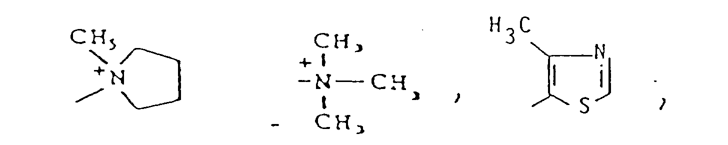
R¹ représente un reste de formule 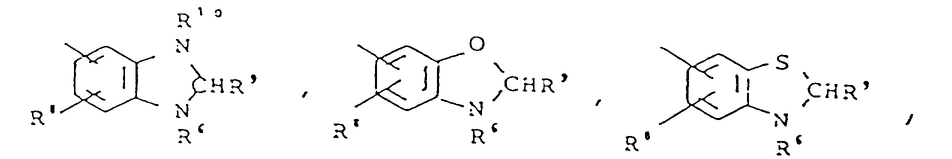 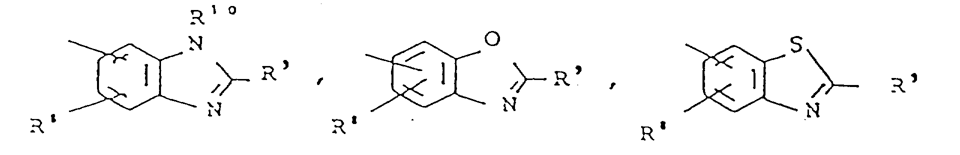 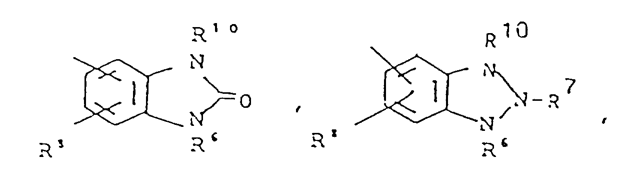 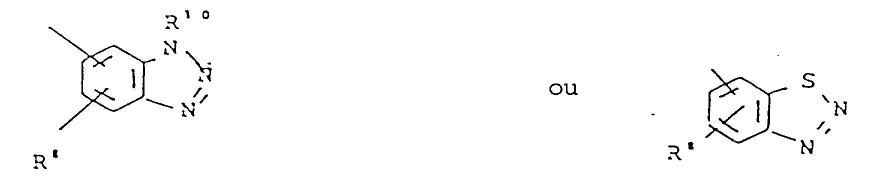
où
R⁶ représente
- de l'hydrogène,
- un groupe hydroxy ou amino, ou
- un groupe alkyle ayant jusqu'à 8 atomes de carbone, linéaire, ramifié ou cyclique, saturé ou non saturé, éventuellement substitué par un ou plusieurs radicaux fluoro, chloro, bromo, amino, hydroxy ou phényle, ou bien
- un groupe phényle,
R⁷
- représente de l'hydrogène ou
- un groupe alkyle ayant jusqu'à 8 atomes de carbone, linéaire, ramifié ou cyclique, saturé ou non saturé, qui est substitué le cas échéant par un ou plusieurs radicaux fluoro, chloro, bromo, alkoxy en C₁ à C₄, hydroxy, carboxy, phényle, SO₃H ou par un groupe amino, ou bien - un groupe phényle,
R⁸ représente
- de l'hydrogène,
- un groupe alkyle, alkoxy, alkylthio ayant chacun 1 à 6 atomes de carbone,
- un groupe trifluorométhyle, trifluorométhoxy,
- un groupe hydroxy, mercapto, nitro, cyano,
- du fluor, du chlore, du brome ou
- un groupe amino,
et dans laquelle
R⁹ a la même définition que R⁷ et représente en outre
- du fluor, du chlore, du brome,
- un groupe alkoxy en C₁ à C₆, alkylthio en C₁ à C₆,
- un groupe amino,
- un groupe -SO₂(alkyle en C₁ à C₆), -PO(OH)₂,
- un groupe -SO₃H ou -SO₂NH₂,
- un groupe SH, OH, S-phényle ou O-phényle,
- un groupe guanidino, -NHNH₂, NHOH, ou
- un groupe pyrrolyle, pyrrolidinyle, pyrazolyle, imidazolyle, pyridyle, pyridazinyle, pyrimidyle, pyrazinyle, quinolyle, isoquinolyle, indolyle, quinoxalyle, quinazolyle, pipéridinyle, oxazolyle, thiazolyle, isoxazolyle, thiadiazolyle, triazolyle ou tétrazolyle portant le cas échéant un ou deux substituants, identiques ou différents, alkyle, alkylthio, alkoxy ayant chacun 1 ou 2 atomes de carbone, fluoro, chloro, bromo, nitro, cyano, hydroxy, amino, trifluorométhyle, trifluorométhoxy ou trifluorométhylthio,
R¹⁰ a la même définition que R⁶, sans toutefois former une double liaison avec R⁷, ou bien
R⁹ et R¹⁰ représentent ensemble une chaîne méthylénique en C₂ à C₄ éventuellement interrompue par du soufre,
R est de l'hydrogène ou
- un groupe protégeant la fonction amino facile à éliminer,
R³ représente
- de l'hydrogène,
- un groupe alkoxy, alkylthio ayant chacun 1 à 3 atomes de carbone,
- un groupe amino ou
- un groupe NHCHO,
et
R⁴ représente
- de l'hydrogène,
- un groupe protégeant la fonction carboxy, classique dans la chimie des β-lactames,
- un groupe -CH₂-O-CO-C(CH₃)₃,
- un groupe -CH(CH₃)-O-CO-O-C₂H₅, -CH₂-O-CO-CH₃,
- le reste de formule 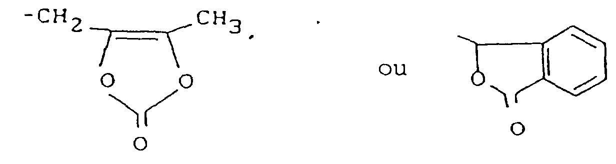 ou
- un ion Na⁺, Li⁺, K⁺ ou NH₄ ⁺.

2. Composés du type du β-lactame suivant la revendication 1, caractérisés en ce que lorsque R est un groupe protégeant la fonction amino, il représente un groupe facilement éliminable, protégeant la fonction amino, choisi dans l'ensemble constitué par un groupe tertiobutoxycarbonyle (Boc), trityle (Trt), benzyloxycarbonyle (Z), formyle, chloracétyle ou 1-méthyl-2-éthoxycarbonylvinyle, ou bien lorsque R⁴ représente un groupe protégeant la fonction carboxy, il s'agit d'un groupe protecteur classique dans la chimie des β-lactames, choisi dans la classe comprenant un groupe tertiobutyle, décyle, 2,2,2-trichloréthyle, benzyle, 4-méthoxybenzyle, 4-nitrobenzyle, tri- ou diphénylméthyle, acétoxyméthyle, allyle ou triméthylsilyle.

3. Procédé de production de composés de la classe des β-lactames de formule générale (I) suivant les revendications 1 et 2, caractérisé en ce qu'on fait réagir des composés de formule générale II 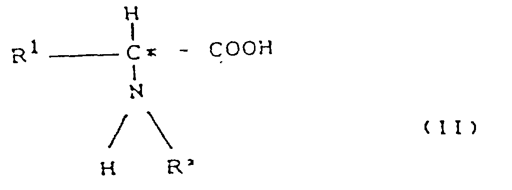 dans laquelle
R¹ a la définition indiquée dans les revendications 1 et 2 et dans laquelle
R est un groupe protégeant la fonction amino,
après activation du groupe carboxyle par transformation en un anhydride mixte, après transformation en le mésylate ou après transformation en un ester activé avec des composés de formule générale (III) 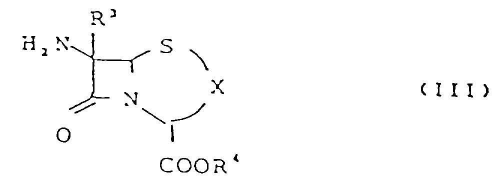 dans laquelle
R³, R⁴ et X ont la définition indiquée dans les revendications 1 et 2, puis le cas échéant, on élimine les groupes protecteurs et on prépare les sels désirés ou les acides libres à partir des sels.

4. Composés du type de β-lactames de formule générale (I) suivant les revendications 1 et 2 et leurs formes D, L et D,L, destinés à être utilisés dans le traitement thérapeutique du corps humain ou animal.

5. Utilisation de composés du type de β-lactames de formule générale (I) suivant les revendications 1 et 2, et leurs formes D, L et D,L, pour la préparation de médicaments.

6. Médicaments contenant des composés du type de β-lactames de formule générale (I) suivant les revendications 1 et 2 et leurs formes D, L et D,L.

7. Aliments pour animaux et mélanges préalables pour aliments pour animaux, caractérisés par une teneur en céphalosporines suivant les revendications 1 et 2.

## Revendications (Revendications pour l'(les) Etat(s) contractant(s) suivant(s): AT)

1. Procédé de production de composés du type de β-lactames de formule générale (I) 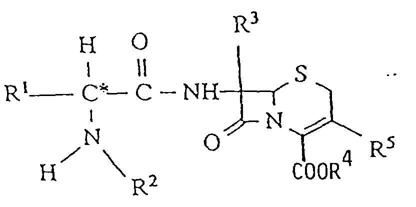 dans laquelle
R⁵ représente
- de l'hydrogène,
- du fluor, du chlore, du brome,
- un groupe alkyle linéaire ou ramifié, saturé ou non saturé, ayant jusqu'à 5 atomes de carbone, qui est substitué le cas échéant par un ou plusieurs radicaux fluoro, chloro, bromo, alkoxy, alkylthio ayant chacun 1 à 3 atomes de carbone, carbamoyloxy, acétyloxy, benzoyloxy ou par un reste de formule 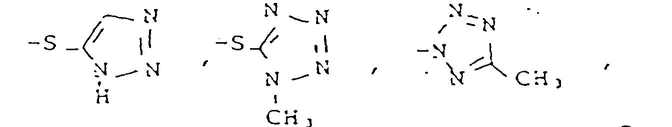 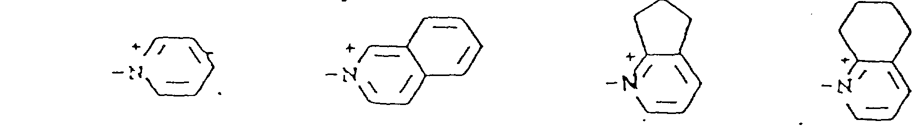 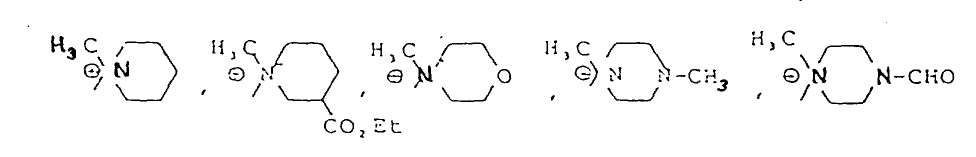 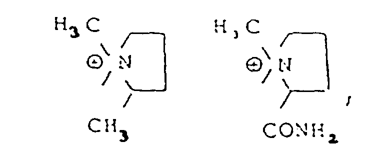 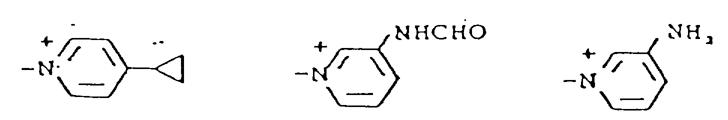 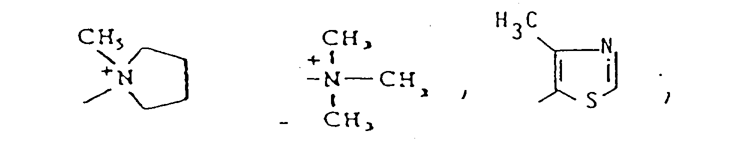
R¹ représente un reste de formule 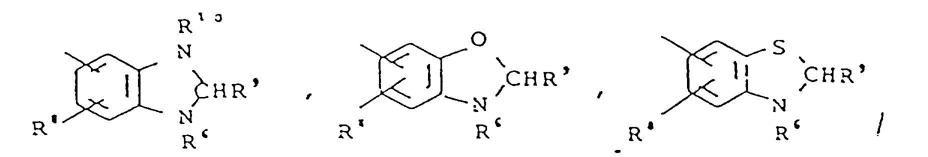 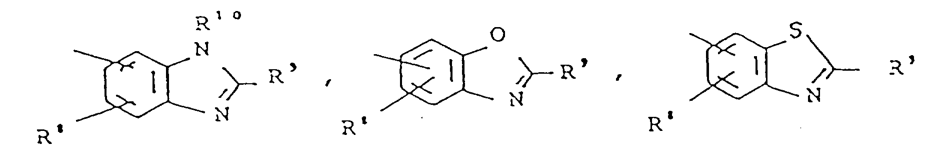 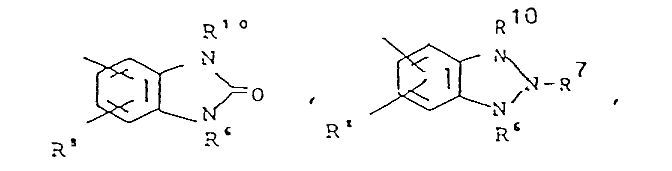 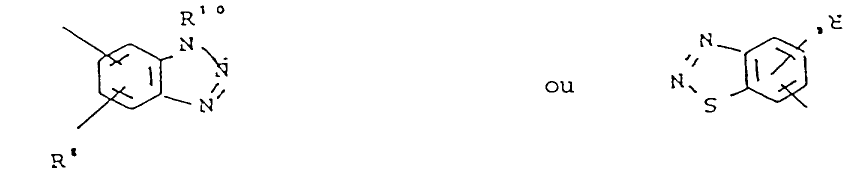
où
R⁶ représente
- de l'hydrogène,
- un groupe hydroxy ou amino, ou
- un groupe alkyle ayant jusqu'à 8 atomes de carbone linéaire, ramifié ou cyclique, saturé ou non saturé, éventuellement substitué par un ou plusieurs radicaux fluoro, chloro, bromo, amino, hydroxy ou phényle, ou bien
- un groupe phényle,
R⁷
- représente de l'hydrogène ou
- un groupe alkyle ayant jusqu'à 8 atomes de carbone linéaire, ramifié ou cyclique, saturé ou non saturé, qui est substitué le cas échéant par un ou plusieurs radicaux fluoro, chloro, bromo, alkoxy en C₁ à C₄, hydroxy, carboxy, phényle, SO₃H ou par un groupe amino, ou bien
- un groupe phényle,
R⁸ représente
- de l'hydrogène,
- un groupe alkyle, alkoxy, alkylthio ayant chacun 1 à 6 atomes de carbone,
- un groupe trifluorométhyle, trifluorométhoxy,
- un groupe hydroxy, mercapto, nitro, cyano,
- du fluor, du chlore, du brome ou
- un groupe amino,
et dans laquelle
R⁹ a la même définition que R⁷ et représente en outre
- du fluor, du chlore, du brome,
- un groupe alkoxy en C₁ à C₆, alkylthio en C₁ à C₆,
- un groupe amino,
- un groupe -SO₂(alkyle en C₁ à C₆), -PO(OH)₂,
- un groupe -SO₃H ou -SO₂NH₂,
- un groupe SH, OH, S-phényle ou O-phényle,
- un groupe guanidino, -NHNH₂, NHOH, ou
- un groupe pyrrolyle, pyrrolidinyle, pyrazolyle, imidazolyle, pyridyle, pyridazinyle, pyrimidyle, pyrazinyle, quinolyle, isoquinolyle, indolyle, quinoxalyle, quinazolyle, pipéridinyle, oxazolyle, thiazolyle, isoxazolyle, thiadiazolyle, triazolyle ou tétrazolyle portant le cas échéant un ou deux substituants, identiques ou différents, alkyle, alkylthio, alkoxy ayant chacun 1 ou 2 atomes de carbone, fluoro, chloro, bromo, nitro, cyano, hydroxy, amino, trifluorométhyle, trifluorométhoxy ou trifluorométhylthio,
R¹⁰ a la même définition que R⁶, sans toutefois former une double liaison avec R⁷, ou bien
R⁹ et R¹⁰ représentent ensemble une chaîne méthylénique en C₂ à C₄ éventuellement interrompue par du soufre,
R est de l'hydrogène ou
- un groupe protégeant la fonction amino facile à éliminer,
R³ représente
- de l'hydrogène,
- un groupe alkoxy, alkylthio ayant chacun 1 à 3 atomes de carbone,
- un groupe amino ou
- un groupe NHCHO,
et
R⁴ représente
- de l'hydrogène,
- un groupe protégeant la fonction carboxy, classique dans la chimie des β-lactames,
- un groupe -CH₂-O-CO-C(CH₃)₃,
- un groupe -CH(CH₃)-O-CO-O-C₂H₅, -CH₂-O-CO-CH₃,
- le reste de formule 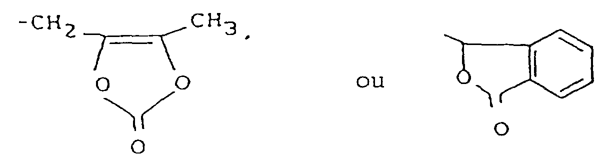 ou
- un ion Na⁺, Li⁺, K⁺ ou NH₄ ⁺,
caractérisé en ce qu'on fait réagir des composés de formule générale (II) 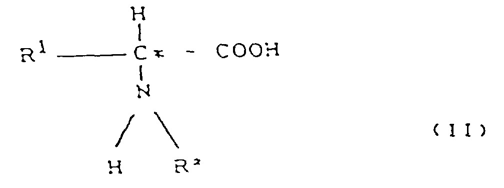 dans laquelle
R¹ a la définition donnée dans la revendication 1 et dans laquelle
R est un groupe aisément éliminable protégeant la fonction amino,
après activation du groupe carboxyle par transformation en un anhydride mixte, après transformation en le mésylate ou après transformation en un ester activé, avec des composés de formule générale (III) 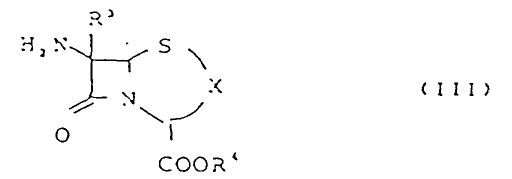 dans laquelle
R³, R⁴ et X ont la définition indiquée ci-dessus, puis on élimine éventuellement les groupes protecteurs et on prépare les sels désirés ou, à partir des sels, les acides libres.

2. Procédé suivant la revendication 1, caractérisé en ce qu'on utilise pour le couplage des amino-acides de formule (II) sur des β-lactames de formule (III) des procédés connus dans la chimie des céphalosporines et des pénicillines.

3. Procédé suivant les revendications 1 et 2, caractérisé en ce qu'on active des amino-acides de formule générale (II) puis on les couple avec des β-lactames de formule générale (III) que l'on dissout sous forme de sels avec une amine.

4. Procédé suivant les revendications 1 et 3, caractérisé en ce qu'on effectue l'activation avec le chlorure d'acide pivalique ou des dérivés d'acides sulfoniques de formule (IV) pour former des anhydrides de formule (V) d'après le schéma 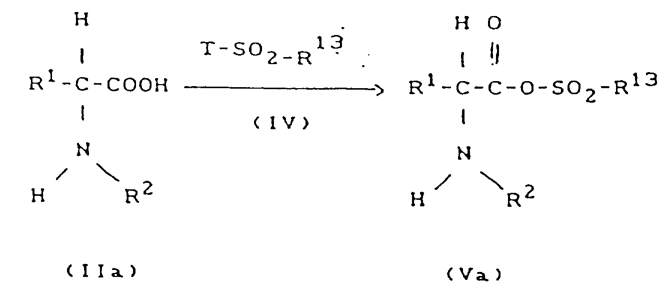 ou 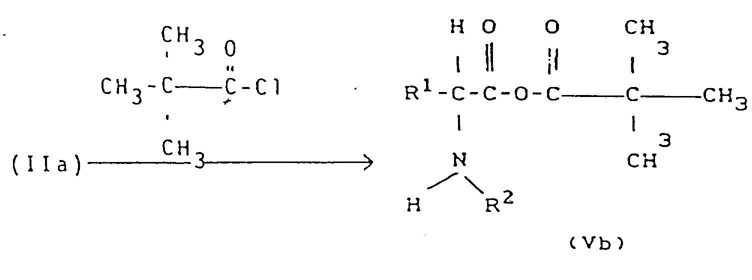 où
R¹ a la définition indiquée dans la revendication 1,
R représente un groupe aisément éliminable protégeant la fonction amino,
T représente un reste R¹³-SO₂-O ou un halogène et
R¹³ est un groupe alkyle en C₁ à C₁₀ qui est substitué le cas échéant par du fluor, du chlore, un radical cyano, phényle, alkyle en C₁ à C₄, alkoxy en C₁ à C₄ ou alkylthio en C₁ à C₄, ou bien
- un groupe phényle éventuellement substitué par un radical fluoro, chloro, bromo, cyano, alkyle en C₁ à C₄, alkoxy en C₁ à C₄, alkylthio en C₁ à C₄, (alkyle en C₁ à C₄)carbonyle, nitro ou trifluorométhyle.

5. Procédé suivant les revendications 1 à 4, caractérisé en ce qu'on conduit les réactions à des températures comprises entre -80°C et à la température ambiante, de préférence entre -60°C et 0°C.

6. Utilisation de composés du type de β-lactames de formule générale (I) suivant les revendications 1 à 5, et de leurs formes D, L ou D,L pour la préparation de médicaments.
